# EUROPEAN PATENT APPLICATION

(11) **EP 2 774 997 A1**
(43) Date of publication of application: **10.09.2014**
(21) Application number: 13157898.1
(22) Date of filing: 05.03.2013
(51) Int. Cl.: C12Q 1/68

(54) **Detection of Androgen Receptor (AR) mutations in circulating tumor DNA from plasma samples of castration-resistant prostate cancer patients using locked nucleic acid-clamp PCR**

(71) Applicant: Bayer Pharma Aktiengesellschaft, 13353 Berlin (DE)
(72) Inventor: Schmitz, Arndt, Berlin (DE); Stresemann, Antje, Berlin (DE); Pieper, Ines Verena, Berlin (DE)
(74) Representative: BIP Patents

(57) **Abstract**

The present invention is directed to a PCR method, novel LNA-blockers and a kit thereof for the detection of nucleic acid mutation(s) in free circulating DNA as present in plasma.

## Description

The present invention is directed to a PCR method, novel LNA-blockers and a kit thereof for the detection of nucleic acid mutation(s) in free circulating DNA as present in plasma.

### Background

Despite the enormous advances made in recent years in our understanding at a molecular level of how cancer develops and progresses and the discovery of potential anticancer agents, more than 90% of these drugs have not been granted a Marketing Authorization. Many drugs fail in the late stages of clinical development (Phase III) because of variable activity in patients, unpredictable safety aspects and inadequate strategies for combating resistance. Hence, there is a need for a more rapid, cost-effective strategy for evaluating anticancer drugs [1, 2]. Identifying patients who will benefit from a particular treatment is still a challenge. However, the difficulty with this lies in the development of suitable clinical tools for discovering indicators or "biomarkers" for cancer [5]. These biomarkers can be divided into a number of different categories, drawing 1. Prognostic biomarkers predict the natural course of tumor growth. They can be used to distinguish between disease courses with a good and poor prognosis. They also help to identify whether or not a patient will benefit from a treatment [5]. Predictive biomarkers assess the likelihood of a patient responding to a particular medication or help identifying patients becoming during the therapy resistant to treatments, prediction and early detection of resistance should prevent unnecessary prescriptions and live space for further therapeutic approaches. Pharmacodynamic (PD) biomarkers, on the other hand, help to establish the optimal dose for a particular drug [2, 5].

The qualitative and quantitative measurement of these biomarkers is another difficulty. Obtaining sufficient tumor biopsy material for biomarker measurement is a major challenge. Tumor biopsy or resection (surgical removal) is typically performed only at the time of diagnosis. Although this seems acceptable for the analysis of prognostic biomarkers, it is limiting in respect of the assessment of predictive biomarkers because these biomarkers are normally measured during the course of treatment [5]. An alternative to re-biopsy is to obtain tumor material using a non-invasive method, e.g. by means of blood sampling. State-of-the-art technologies have therefore been established such as circulating tumor cell (CTC) analysis [8, 9], the performance of mutation-specific PCR assays using circulating tumor DNA (ctDNA) [10] and plasma/serum proteome analysis. Non-invasive technologies also include in situ tumor analysis at a molecular level and the use of specific autoantibodies against tumor cells [11].

### Circulating tumor DNA (ctDNA):

Cell-free circulating tumor DNA (ctDNA) in the plasma, serum, urine or stool can also be used as a liquid biopsy. Changes in ctDNA levels have been associated with tumor burden and malignant progression. In recent years, many attempts have been made to demonstrate the potential use of ctDNA for identifying therapeutic targets. ctDNA was expected to be used for identifying resistance to certain therapeutic agents due to specific mutations [61].

### Circulating tumor DNA (ctDNA) a clinical biomarker:

The presence of circulating free DNA (cfDNA) in human blood from subjects was first described in 1948 by Mandel and Métais [62]but it was not until 1994 that ctDNA was discovered in the blood of tumor patients (mutated Ras gene fragments) and associated with malignant tumor progression. Approximately 3.3% ctDNA will be released per day from a tumor weighing 100 g (approximately 3 x 10¹⁰ tumor cells) [61]. Circulating free (tumor) nucleic acids are usually present in the plasma or serum in the form of short fragments of between 70 and 200 bp in length. Very large fragments of up to 21 kb in length have also been detected, [10, 63]. The concentration of cfDNA in biological fluids is usually very low and varies greatly between individuals within a range of 1-100 ng/ml [64]. A correlation has nevertheless been confirmed between high cfDNA concentrations (possibly increased by ctDNA) in tumor patients and low concentrations in subjects [65]. In early tumor development, ctDNA is shed passively by the primary tumor into the bloodstream via apoptotic and/or necrotic processes. Necrotic or apoptotic cells are normally phagocytized by macrophages. Macrophages that digest necrotic cells may release this DNA into surrounding tissue. *In vitro* cell culture experiments have shown that macrophages are either activated during this process or die off during DNA release [65, 66]. The active release of tumor cells may itself be an additional mechanism for the release of ctDNA, drawing 3. Besides, the mechanisms for ctDNA formation described above, Fleischhacker and Schmidt (2007) published that ctDNA can also be released as a consequence of inflammatory processes during tumor progression in tumor patients [67]. In addition, ctDNA levels are also determined by the rate of degradation by DNases and by physiological filtering of the blood. Nucleic acids in the blood are filtered by the liver and kidneys and have a half-life ranging between 15 minutes and several hours in circulating blood [67]. Bendich et *al.* (1965) published that double-strand ctDNA was detected for longer in circulating blood than single-strand ctDNA [68]. An excess of dying cells, as often observed in tumors, leads to saturation of the process and to an increased concentration of ctDNA in the blood [66]. In the peripheral blood, this ctDNA circulates primarily in the form of mono- or oligonucleosomes (this first chromatin packaging step of higher cells is a complex of DNA and histones) or is bound to DNA-binding proteins on the surface of blood cells [66,69]. This ctDNA can be isolated from the blood and used for measuring genetic and/or epigenetic parameters [65]. ctDNA analysis provides a means of demonstrating tumor progression at a molecular level and of documenting the mutational status of the tumor. The detection of mutations in tumor tissue has an influence on disease prognosis and choice of treatment. It is necessary both to know the mutational status of the tumor at diagnosis and to detect newly acquired mutations during the course of treatment. In this connection, it has been possible, using quantitative methods of measurement, to establish a correlation between ctDNA levels and tumor size and stage and/or a poor prognosis [70],presenting an opportunity for monitoring patients longitudinally during treatment and for predicting (non-)response to a medication [65]. There is also a strong correlation between the ctDNA mutation data and the mutational status of the primary tumor [71]. At present time, mutational status testing is still often performed using formalin-fixed paraffin-embedded material. A major problem when it comes to detecting the mutational status of a tumor is the lack of specificity and sensitivity of the detection methods. For the performance of ctDNA mutation analyses, a large quantity of ctDNA must be present in blood. ctDNA is typically to be found in the presence of a high background of wild-type, circulating DNA, which makes analysis difficult. Although a lot of evidence exists of the prognostic and predictive relevance of ctDNA as a biomarker, there have also been publications which have shown no correlation at all between the accumulation of ctDNA and tumor progression [66, 72].

Analytical methods for the sensitive detection of point mutations in ctDNA: In order to detect mutated DNA by means of Sanger sequencing in a wild-type (WT) background, the mutated DNA must account for at least 25% of the total DNA content [73]. In clinical samples, the proportion of mutated DNA is between 100 and 10,000 times smaller than thiswhich means that mutation detection is impossible using conventional methods. Analytical methods are available for quantifying the concentration of wild-type and mutated DNA simultaneously. It was observed that some tumor patients develop resistance during drug treatment because of mutations, with individual SNPs (single nucleotide polymorphisms) often being the cause of resistance to a particular therapy. Some of the sensitive analytical methods for detecting SNPs are summarized below for illustration purposes.

### LNA clamp PCR:

With the aid of LNA clamp PCR, enrichment of the mutated DNA sequence to be analyzed occurs during the PCR itself. Amplification of the Wild-type (WT) sequence can be suppressed in two ways.
Either the amplification of the WT by binding of the primer to the WT [79,80] or the inhibition of amplification of the WT, drawing 4) [81,82]. In both cases, an oligonucleotide is designed which has 100% complementarity to the WT sequence. LNAs (locked nucleic acids) nucleotide analogues [83, 84] are used as blockers. These nucleotide analogues have the property of binding to the complementary WT sequence with a high degree of affinity. Achievable detection limits of 1:100-1:10,000 are described [83_85]. One drawback of this method is the relatively high consumables cost of the blockers. The risk of false-positive products, as associated with allele-specific PCR, is largely eliminated.

Obika et *al.* (1997) [87] designed 'LNA' oligonucleotides at almost the same time as Koshkin et *al.* (1998) [88]. The Danish biotech company EXIQON is specialized into the design and production of 'LNA' oligonucleotides since 1997. LNAs consist of modified RNA nucleotides wherein the LNA monomer consists of a bicyclic nucleic acid in which the ribose subunit has been modified with an additional methyl group. This connects the 2' oxygen atom to the 4' carbon atom. This covalent bond 'locks' ribose in the 3'-endo (North) conformation which occurs mainly in the A-form of DNA or RNA, drawing 5. This conformation is ideal for Watson-Crick binding. Without this bridge, the ring may oscillate between the North and South conformation. The 'locked' conformation enhances base stacking because the LNA modification stabilizes the water structure in the vicinity of the methylene bridge and thus also the DNA duplex. For this reason, complementary DNA or RNA is bound with much higher affinity. Thermal stability is also enhanced. For each incorporated LNA monomer, the melting temperature (Tm) increases by approximately 8 C. LNA monomers also display nuclease resistance (endo-, exonuclease) [89]. As a result of this altered thermal stability, LNA oligomers are able to achieve greater sensitivity and specificity than conventional DNA oligomers, especially in relation to the discrimination of WT and mutated DNA fragments containing individual base mutations [89].

Amplification of the WT fraction is suppressed as a result of further synthesis by the DNA polymerase being inhibited ('locked'/'clamped') once the blocker is reached. Amplification of the mutated fraction continues unhindered because the blocker is bound more weakly as a result of the base-base mispairing and becomes detached again during elongation. The 100% complementarity of the LNA probe binding to the WT is referred to as 'perfect match' and the weaker binding of the probe to the mutation as a result of the base-base mispairing as 'mismatch'. As a result of the perfect base pair match between the probe and the WT, the Tm of the perfect match is higher than that of the mismatch. This difference in temperature is increased in the probe compared with normal deoxynucleotides (maximum dTm 0.5-3 C) by the incorporation of LNA monomers. The higher the probe dTm between perfect match and mismatch, the better the discrimination of the mutation in the WT background [89].

Oldenburg et al., Journal of Investigative Dermatology (2008) 128, 398-402 describe an approach to detect circulating melanoma cells harboring a common point mutation in the BRAF kinase. In the first step, primer binding to wild-type BRAF is competitively blocked by a locked nucleic acid (LNA) oligonucleotide. In the second step, the LNA-blocking approach is combined with a mutant-specific forward primer. To determine the clinical utility of this method, they tested its ability to detect human blood spiked with a defined number of BRAF1799T4A-mutated melanoma cells as a surrogate for real patient samples. Blood was first enriched for melanoma cells using an antibody-mediated negative selection procedure before whole genome amplification (WGA). Mutant BRAF in the WGA-amplified genomic DNA was further amplified by a two-step real-time PCR protocol. The method is here directed to circulating cells and to DNA isolated from circulating cells.

### Description of drawings:

Drawing 1: Various functions of biomarker-based tests. Prognostic biomarkers predict the natural course of tumor growth. Their purpose is to identify the need for medication. Predictive biomarkers identify substances to which a patient is likely to respond. Pharmacodynamic biomarkers help to establish the optimal dose. Diagram taken from [7].
Drawing 3: Source of circulating tumor DNA. Large quantities of ctDNA circulate in the blood of tumor patients. This ctDNA could have been released either from a primary tumor or (micro-) metastases or from apoptotic circulating tumor cells (2). The ctDNA can be extracted from the blood and genetic and/or epigenetic changes can be determined. Diagram taken from [65].
Drawing 4: LNA blocker as an inhibitor of amplification. By binding to the wild-type fragment, the blocker inhibits polymerase-induced chain elongation. Mutant amplification can continue unhindered as a result of the mispairing with the LNA blocker. Diagram taken from [86].
Drawing 5: The chemical structure of an LNA monomer. An LNA monomer contains a ribonucleotide which is closed by means of a methylene bridge between the 2' oxygen atom and the 4' carbon atom. The bicyclic nucleotide is thus forced into a 3'-endo conformation. The diagram on the left illustrates the chemical structure. The diagram on the right illustrates the 'locked' 3'-endo conformation of the furanose ring. Diagram taken from [90].
Drawing 6: Schematic representation of the mRNA sequence of the human AR.
   The diagram shows the 4,314 bp long mRNA sequence of the human androgen receptor (RefSeq: NM000044). 5' UTR (untranslated region) and 3' UTR flank the ORF (open reading frame, grey brackets). The mRNA consists of exons A to H and codes for AR isoform I. The region of exon A identified with a '1' codes for the transactivation domain. Exon B and exon C code for the DNA-binding domain ('2'). The region between exon D and exon H codes for the ligand-binding domain ('3'). The W741C (exon E) and T877A (exon H) hotspots are located in this region (grey frame). Drawing 7: Schematic representation of the genomic DNA region containing the T877A point mutation in the human AR. The diagram shows exon H of the WT and mutant gDNA sequence of the human AR. Primer AR1_FOR_T877A and primer AR5_REV_T877A amplify the 259 bp long AR_T877A fragment. Primer AR1_FOR_T877A was designed so that it attached to the template strand just a few bases upstream of the blocker. The LNA blocker binds complementarily to the 16 nt long WT sequence in which codon 877 is located. It consists of a mixture of LNA monomers (identified with a '*') and deoxynucleotides. Codon 877 is highlighted in turquoise. The base substitution (A -> G) is highlighted in red in codon 877. The amino acid is annotated above the WT and mutation sequence. Drawing 8: Schematic representation of the genomic DNA region containing the W741C point mutation in the human AR. The diagram shows exon E of the WT and mutant gDNA sequence of the human AR. Primer AR1_FOR_W741C and primer AR5_REV_W741C amplify the 279 bp long AR_W741C fragment. Primer AR1_FOR_W741C was designed so that it attached to the template strand just a few bases upstream of the blocker. The LNA blocker binds complementarily to the 10 nt long WT sequence in which codon 741 is located. It consists of a mixture of LNA monomers (identified with a '+') and deoxynucleotides. Codon 741 is highlighted in turquoise. The base substitution (G -> T) is highlighted in dark grey in codon 741. The amino acid is annotated above the WT and mutation sequence.
Drawing 9: Schematic representation of the genomic DNA region containing the W741C point mutation in the human AR. The diagram shows exon E of the genomic DNA sequence of the human AR (highlighted in yellow). Primer AR1_FOR_W741C and primer AR5_REV_W741C (grey letters) amplify the 279 bp long AR_W741C fragment. Primer AR5_REV_W741C is located in the adjoining intron. The coding region (see reading frame +3) is represented by the amino acid sequence. The LNA™ clamp binds complementarily to a 10 nt long sequence (dark grey letters) in which codon 741 is located (highlighted in pink). The base substitution (G to T) is highlighted in dark grey in codon 741. Drawing 10: Schematic representation of the genomic DNA region containing the T877A point mutation in the human AR. The diagram shows exon H of the genomic DNA sequence of the human AR (highlighted in yellow). Primer AR1_FOR_T877A and primer AR5_REV_T877A (blue letters) amplify the 259 bp long AR_T877A fragment. Primer AR1_FOR_T877A is partially located in the intron. The coding region (see reading frame +1) is represented by the amino acid sequence. The 3' UTR region of exon H comes after the stop codon (***). The LNA™ clamp binds complementarily to a 16 nt long sequence (dark grey letters) in which codon 877 is located (highlighted in pink). The base substitution (A to G) is highlighted in dark grey in codon 877.
Drawing 11: Optimization of the LNA concentration of the T877A batch.
Drawing 12: Optimization of the LNA concentration of the W741C batch.
Drawing 13: Determination of the mutant DNA detection limit in the presence of an excess of WT DNA using standard dilutions.
Drawing 14: Sequencing results for W741C/T877A-LNA batches in standard dilutions.
Drawing 15: Determination of Bicalutamide resistance of clinical samples using W741C-LNA blockers.
Drawing 16: Sequencing results for the clinical samples for the identification of Bicalutamide resistance.
Drawing 17: 3' Inverted Thymidine (3InvdT).
Drawing 18: Individual case report for patient 5. The diagram shows the development of the prostate cancer of the patient over time as indicated by his PSA levels. PSA values were determined in a clinical routine lab using the Hybritech assay (Beckman-Coulter). Patient was treated with Bicalutamide from Oct. 2008 to Sept. 2009 when (after initial return to normal) the PSA level increased under therapy indicating a clinical diagnosis of castration resistance. Blood used to obtain plasma for PCR analysis was drawn in Nov. 2011, 26 months after end of Bicalutamide therapy.

### Problem and Solution

Patients with advanced prostate cancer are often treated with the aid of antiandrogens such as Bicalutamide or Flutamide. After a period of treatment, urologists identify the presence of castration-resistant prostate cancer on the basis of an increase in the PSA value and a general deterioration in the clinical picture. This is attributed to tumor cell resistance to antiandrogen therapy. The two point mutations T877A/W741C in the human AR are considered to be a key factor in resistance to treatment with Bicalutamide and Flutamide respectively. The standard methods, as described above, for analyzing point mutations or SNPs achieve detection limits within the range of 1:100-1:1,000, and at 1:10,000 in rare cases. The main challenge in terms of detection lies in the ratio of mutated to non-mutated DNA fragments.

The present invention provides an improved LNA clamp qRT-PCR method wherein ctDNA is detectable in patient plasma with a reasonable detection limit.

### Description

In a first aspect, the invention is directed to a PCR method for detecting the presence of at least one nucleotide Mutation in DNA polynucleotide sequence encoding for a protein or fragments thereof isolated from patient plasma, characterized in that
- A LNA-blocker is used and
- A DNA polynucleotide sequence or fragments thereof is a natural circulating tumor DNA.

Preferred Features:
The PCR method comprises the steps of
   1. Denaturing the DNA polynucleotide sequences,
   2. Annealing the DNA polynucleotide sequences with a LNA-blocker, an upstream primer and a downstream primer,
   3. Elongating the DNA polynucleotide sequence in presence of a polymerization agent,
   4. Optionally repeating Denaturing, Annealing and Elongation steps as necessary, and
   5. Detecting amplified DNA polynucleotide sequences.

Preferably, the PCR method is a LNA clamp qRT-PCR.

DNA polynucleotide sequences encoding for a protein are well known for a large number of proteins as well as the amino acid and nucleotide mutations resulting into dysfunction of the protein. For example, Androgen Receptor (AR) genes were sequenced and gene mutations thereof were identified. Such gene mutations are potential target for developing novel treatment. AR is important in the development and progression of prostate cancer. It was observed that in some patients with anti-Androgen Receptor drugs might lead to a resistance and decrease of the hormonal therapy efficiency leading to castration-resistant prostate cancer (CRPC). Despite an initial favorable response, patients progress to a more aggressive, castrate-resistant phenotype that is characterized by the presence of certain mutations of the Androgen Receptor (AR) gene in the circulating tumor cells (CTCs).

### Encoded protein:

Preferably, the DNA polynucleotide sequence is encoding for an Androgen Receptor (AR) or fragments thereof.

More preferably, the DNA polynucleotide sequence is encoding for an Androgen Receptor (AR) or fragments thereof with Mutation at the amino acid position 741 and/or 877.

Even more preferably, the DNA polynucleotide sequence is encoding for an Androgen Receptor (AR) or fragments thereof with Mutation at the amino acid position 741 and/or 877 resulting into patient with castration-resistant prostate cancer (CRPC).

Even more preferably, the DNA polynucleotide sequence is encoding for an Androgen Receptor (AR) or fragments thereof with Mutation at the amino acid position 741 and/or 877 resulting into patient with castration-resistant prostate cancer (CRPC) being treated with Bicalutamide or Flutamide.

The invention is directed to Androgen Receptor (AR) Mutation at the amino acid position 741 wherein the Wild amino acid Threonine (Thr) is replaced by Alanine (Ala). (Thr [Wild] => Ala [Mut]). The invention is directed to Androgen Receptor (AR) Mutation at the amino acid position 877 wherein the Wild amino acid Tryptophan (Trp) is replaced by Cysteine (Cys). (Trp [Wild] => Cys [Mut]).

The DNA polynucleotide sequence encoding for Homo sapiens Androgen Receptor (AR) is fully documented under the reference Sequence: NG_009014.2 of the National Center for Biotechnology Information (NCBI).

The mRNA sequence of the Homo sapiens Androgen Receptor (AR), transcript variant 1, is documented under the reference Sequence: NM_000044.3 of the National Center for Biotechnology Information (NCBI).

The protein sequence of the Homo sapiens Androgen Receptor (AR), isoform 1 is documented under the reference Sequence: NP_000035.2 of the National Center for Biotechnology Information (NCBI).

**DNA polynucleotide sequence** is isolated from patient plasma and corresponds to circulating free DNA comprising circulating tumor DNA (ctDNA) from tumor cells and further circulating DNA from healthy cells. The DNA Polynucleotide sequence is mostly in the form of short or long fragments. Preferably, the DNA Polynucleotide sequence comprises at least one nucleotide Mutation. More preferably, the DNA Polynucleotide sequence comprises at least one nucleotide Mutation wherein said Mutation lead to an amino acid Mutation of the encoded protein.

The DNA Polynucleotide sequence comprises at least one nucleotide Mutation means that at least one nucleotide of the DNA polynucleotide sequence was replaced by another nucleotide. The nucleotide replacement is a mutation of the DNA polynucleotide sequence.

"At least one nucleotide Mutation" means preferably 1 to 5 Mutations. "At least one nucleotide Mutation" means more preferably, 1 or 2 Mutations, even more preferably 1 Mutation. Preferably, the Mutation is characteristic for patient showing a resistance to certain therapy drugs. More preferably, the Mutation is characteristic for patient showing a resistance to Bicalutamid or Flutamide.

More preferably, the DNA polynucleotide sequence presents Mutation in nucleotide codons 741 and/or 877 of the Androgen Receptor (AR) tumor DNA. Even more preferably, the DNA polynucleotide sequence presents Mutation in nucleotide codons 741 and/or 877 of the Androgen Receptor (AR) tumor DNA wherein Mutation corresponds to a single nucleotide base change. Preferably, the Mutation is the replacement of Adenine (A) with Guanine (G) in the 1st position of the codon corresponding to amino position 877.

Preferably, the Mutation is the replacement of Guanine (G) with Thymine (T) in the 3^{rd} position of the codon 741.

DNA AR Codon W741C (Exon E):
Wild => Mutation
_{5'}T-G-G_{3'} => _{5'}T-G-T_{3'}

DNA AR Codon T877A (Exon H):
Wild => Mutation
₅,A-C-T_{3'} => _{5'}G-C-T_{3'}

Drawing 6 shows the position of Mutations T877A and W741C.

**Upstream and downstream primers** are short oligonucleotide (10 to 25 nucleic bases) complementing/hybridizing specifically to the 5' and 3' extremity, respectively, of the DNA oligonucleotide sequence encoding for a polypeptide or a protein.
Upstream primers and downstream primers are preferably

| | |
|---|---|
| SEQ ID 1:AR1_FOR_W741C | GACCAGATGGCTGTCATTCA |
| SEQ ID 2: AR5_REV_W741C | AAAGCCGCCTCATACTGGAT |
| SEQ ID 3: AR1_FOR_T877A | CCCTACAGATTGCGAGAGAG |
| SEQ ID 4: AR5_REV_T877A | CAAGGCACTGCAGAGGAGTA |

The **LNA-blocker** is defined as a LNA-oligonucleotide sequence of 6 to 25 bases comprising a mixture of nucleic acid bases and LNA-monomer bases wherein the LNA-oligonucleotide sequence is complementing/hybridizing a specific DNA sequence of a DNA oligonucleotide sequence encoding for a protein or fragments thereof or a DNA target gene wherein the specific DNA sequence is a Wild type DNA sequence for which one or more Mutations were identified and found related to none-response to a pharmaceutical treatment. In other words, the LNA-blocker is complementing a Wild type DNA sequence that is selected because of identified mutation(s) within this sequence. These Mutations are qualifying as a potential biomarker for identifying patient not-responding to a given pharmaceutical treatment.

Preferably, the LNA-oligonucleotide sequence is encoding for a protein wherein nucleotide mutations thereof results into dysfunction of the encoded protein.

More preferably, the LNA-oligonucleotide sequence is encoding for an Androgen Receptor (AR) or fragments thereof wherein nucleotide mutations thereof results into patient suffering from castration-resistant prostate cancer (CRPC).

Preferably, the Wild type DNA sequence is defined as such that one Mutation was identified within this sequence and found related to none-response to a pharmaceutical treatment. More preferably, the Wild type DNA sequence is defined as such that one Mutation was identified within this sequence and found related to none-response to a pharmaceutical treatment thereof due to dysfunctionment of the encoded protein. Even more preferably, the Wild type DNA sequence is defined as such that one Mutation was identified within this sequence and found related to none-response to a pharmaceutical treatment thereof due to dysfunctionment of the encoded protein and resulting into castration-resistant prostate cancer (CRPC) wherein the pharmaceutical treatment consists into administration of Flutamide or Bicalutamide to a patient in need.
Preferably, the Mutation is defined as above.

Preferably, the LNA-blocker is defined as a LNA-oligonucleotide sequence of 6, 7, 8, 9, 10, 11, 12, 13, 14, 15, 16, 17, 18, 19 or 20 bases comprising a mixture of nucleic acid bases and LNA-monomer bases. More preferably, the LNA-blocker is defined as a LNA-oligonucleotide sequence of bases comprising a mixture of 10 to 18 or 11 to 17 nucleic acid bases and LNA-monomer bases. Even more preferably, the LNA-blocker is defined as a LNA-oligonucleotide sequence of 11 or 17 bases, independently from each other, comprising a mixture of nucleic acid bases and LNA-monomer bases. Even more preferably, the LNA-blocker is defined as a LNA-oligonucleotide sequence of 11 bases selected from SEQ ID 5: W741C_LNA.

Even more preferably, the LNA-blocker is defined as a LNA-oligonucleotide sequence of 17 bases selected from SEQ ID 6: T877A_LNA.

In **first embodiment,** the LNA-blocker comprises 1 to 12, 2 to 12 or 3 to 12 LNA-monomer bases with the proviso that the LNA-blocker comprises at least 1 nucleic acid base. Preferably, the LNA-blocker comprises 7 to 11 LNA-monomer bases. More preferably, the LNA-blocker comprises 8, 9 or 10 LNA-monomer bases.

Even more preferably, the LNA-blocker comprises 8 LNA-monomer bases and is corresponds to SEQ ID 5: W741C_LNA.

Even more preferably, the LNA-blocker comprises 10 LNA-monomer bases and is corresponds to SEQ ID 6: T877A_LNA.

In **second embodiment,** the LNA-blocker is defined as beginning [5'] and ending [3'] with a LNA-monomer base.

Preferably, the LNA-blocker is defined as a LNA-oligonucleotide sequence of 10 bases selected from SEQ ID 7: W741C_LNA_np.

Preferably, the LNA-blocker is defined as a LNA-oligonucleotide sequence of 16 bases selected from SEQ ID 8: T877A_LNA_np.

In third embodiment, the LNA-blocker is defined as having a LNA-monomer base positioned in the central portion of the LNA-oligonucleotide sequence. Preferably, the LNA-blocker is defined having a LNA-monomer base positioned in the central portion of the LNA-oligonucleotide sequence wherein said LNA-monomer base is at the position of the Mutation.

Central portion of the LNA-oligonucleotide sequence means that the LNA-monomer base complementing the Mutation to be detected is located in the middle +/- 2 nucleotide of the LNA-oligonucleotide sequence.

The LNA-monomer base at the position of the Mutation corresponds to the Wild type nucleic acid base.

More preferably, the LNA-blocker is defined as SEQ ID 5: W741C_LNA having a LNA-monomer base G*₆ at the Mutation position.

More preferably, the LNA-blocker is defined as SEQ ID 6: T877A_LNA having a LNA-monomer base A*₇ at the Mutation position.

In a **fourth embodiment,** the LNA-blocker is carrying a protecting group defined as a 3' Inverted Thymidine (3InvdT) at the 3' extremity.
Preferably, the LNA-blocker is SEQ ID 5: W741C_LNA comprising a 3InvdT.
Preferably, the LNA-blocker is SEQ ID 6: T877A_LNA comprising a 3InvdT.

In a **fifth embodiment,** the LNA-blocker is defined as
- the LNA-oligonucleotide sequence of 6 to 25 bases comprising a mixture of nucleic acid bases and LNA-monomer bases wherein the LNA-oligonucleotide sequence is complementing/hybridizing a DNA polynucleotide sequence at a specific DNA sequence of a DNA polynucleotide sequence encoding for a protein or fragments thereof or a DNA target gene wherein the specific DNA sequence is a Wild type DNA sequence for which one or more Mutations were identified and found related to none-response to a pharmaceutical treatment,
- the LNA-oligonucleotide sequence comprises 1 to 12, 2 to 12 or 3 to 12 LNA-monomer bases, the LNA-oligonucleotide sequence is beginning and ending with a LNA-monomer base and
- the LNA-blocker is carrying a protecting group defined as an 3' Inverted Thymidine (3InvdT).

Preferably, the LNA-blocker is SEQ ID 5: W741C_LNA of 11 bases complementing/hybridizing a DNA at the specific position of the codon 741 of AR wherein the LNA-blocker
comprises 8 LNA-monomer bases,
begins with T* LNA-monomer base,
ends G* LNA-monomer base and
carries a protecting group defined as an 3' Inverted Thymidine (3InvdT).

Preferably, the LNA-blocker is SEQ ID 6: T877A_LNA of 17 bases complementing/hybridizing a DNA at the specific position of the codon T877A of AR wherein the LNA-blocker
comprises 10 LNA-monomer bases,
begins with C* LNA-monomer base,
ends C* LNA-monomer base and
carries a protecting group defined as an 3' Inverted Thymidine (3InvdT).

Preferably, the LNA-blocker is SEQ ID 7: W741C_LNA_np.

Preferably, the LNA-blocker is SEQ ID 8: T877A_LNA_np.

| LNA-Blockers | |
|---|---|
| SEQ ID 5: W741C_LNA | T*-C*-C*-**T**-G*-G*-**A**-T*-G*-G*/3InvdT/ |
| | T*-C*-C*-**T**-G*-G*-**A**-T*-G*-G*/3InvdT/ |
| | with highlighted LNA-monomer position of the nucleotide Mutation |
| | T*₁-C*₂-C*₃-**T**₄-G*₅-G*₆-**A**₇-T*₈-G*₉-G*₁₀/3InvdT₁₁/ |
| | with nucleotide position numbering |
| SEQ ID 6: T877A_LNA | C*-A*-G*-**T-T**-C*-A*-C*-T*-**T-T-T**-G*-**A**-C*-C*/3InvdT/ |
| | C*-A*-G*-**T-T**-C*-A*-C*-T*-**T-T-T**-G*-**A**-C*-C*/3InvdT/ |
| | with highlighted LNA-monomer position of the nucleotide Mutation |
| | C*₁-A*₂-G*₃-**T**₄-**T**₅-C*₆-A*₇-C*₈-**T***₉-**T**₁₀-**T**₁₁-**T**₁₂-G*₁₃-A₁₄-C*₁₅-C*₁₆/3InvdT₁₇/ |
| | with nucleotide position numbering |
| SEQ ID 7: W741C_LNA_np | T*-C*-C*-**T**-G*-G*-**A**-T*-G*-G* |
| SEQ ID 8: T877A_LNA_np | C*-A*-G*-**T-T**-C*-A*-C*-T*-**T-T-T**-G*-**A**-C*-C* |

| | |
|---|---|
| "3InvdT" means 3' Inverted Thymidine. "np" means non-protected. "A*", "G*", "C*" and "T*" stand for LNA-monomer base. A, G, C, and T stand for natural and synthetic nucleic acid base. | |

DNA stands for genomic DNA and fragments thereof.

LNA-monomers are described above and in drawing 5.

The annealing step is conducted at a temperature of 52 to 60 C°. Preferably, the annealing step is conducted at a temperature of 54 to 58 C°.

Preferably, when the method for detecting a Mutation is directed to the Mutation in the codon corresponding to the amino acid 741of AR, the LNA blocker is defined as SEQ ID 5: W741C_LNA, the upstream primer is defined as SEQ ID 1: W741C, the downstream primer is defined as SEQ ID 2: AR5_REV_W741C then the annealing step is conducted at a temperature of 55 C°.

Preferably, when the method for detecting a Mutation is directed to the Mutation in the codon corresponding to the amino acid 877 of AR, the LNA blocker is defined as SEQ ID 6: T877A_LNA, the upstream primer is defined as SEQ ID 3: the T877A, downstream primer is defined as SEQ ID 4: AR5_REV_T877A then the annealing step is conducted at a temperature of 60 C°.

The elongating step is conducted at a temperature of 72 to 74 C°.

Preferably, when the method for detecting a Mutation is directed to the Mutation in the codon corresponding to the amino acid 741 of AR, the LNA blocker is defined as SEQ ID 5: W741C_LNA, the upstream primer is defined as SEQ ID 1: W741C, the downstream primer is defined as SEQ ID 2: AR5_REV_W741C then the elongating step is conducted at a temperature of 74 C°.

Preferably, when the method for detecting a Mutation is directed to the Mutation in the codon corresponding to the amino acid 877 of AR, the LNA blocker is defined as SEQ ID 6: T877A_LNA, the upstream primer is defined as SEQ ID 3: T877A, the downstream primer is defined as SEQ ID 4: AR5_REV_T877A then the elongating step is conducted at a temperature of 72 C°.

### Preferred PCR method:

Preferably, the method is for detecting the presence of at least one nucleotide Mutation in a DNA polynucleotide sequence encoding for a protein or fragments thereof in patient plasma wherein a patient with a Mutation is a patient showing a resistance to certain therapy drugs. Preferably, the patient with a Mutation is a patient showing a mutation in AR. Said patient shows a resistance to certain therapy drugs and developing a castration-resistant prostate cancer (CRPC). More preferably, the patient with a Mutation is a patient showing a resistance to therapy drugs such as Bicalutamide and/or Flutamide and developing a castration-resistant prostate cancer (CRPC).

Preferably, the PCR method is for detecting the presence of at least one nucleotide Mutation in a DNA polynucleotide sequence encoding for a Androgen Receptor (AR) or fragments thereof isolated from patient plasma,
characterized in that
- A LNA-blocker is used and
- A DNA polynucleotide sequence or fragments thereof is a natural circulating
   tumor DNA.

More preferably, the PCR method is for detecting the presence of at least one nucleotide Mutation in a DNA polynucleotide sequence encoding for an Androgen Receptor (AR) or fragments thereof with Mutation at the amino acid position 741 and/or 877 isolated from patient plasma, characterized in that
- A LNA-blocker is used and
- A DNA polynucleotide sequence or fragments thereof is a natural circulating tumor DNA,
wherein the LNA-blocker is a LNA-oligonucleotide sequence of 6 to 25 bases comprising a mixture of nucleic acid bases and LNA-monomer bases wherein the LNA-oligonucleotide sequence is complementing/hybridizing a specific DNA sequence of a DNA polynucleotide sequence encoding for an Androgen Receptor (AR) or fragments thereof wherein the specific DNA sequence is a Wild type DNA sequence for which one or more Mutations were identified and found related to none-response to a pharmaceutical treatment.

Even more preferably, the PCR method is for detecting the presence of at least one nucleotide Mutation in a DNA polynucleotide sequence encoding for an Androgen Receptor (AR) or fragments thereof isolated from patient plasma, characterized in that
- A LNA-blocker is SEQ ID 5: W741C_LNA and
- A DNA polynucleotide sequence or fragments thereof is a natural circulating
   tumor DNA.

Even more preferably, the PCR method for detecting the presence of at least one nucleotide Mutation in a DNA polynucleotide sequence encoding for an Androgen Receptor (AR) or fragments thereof isolated from patient plasma,
characterized in that
- A LNA-blocker is SEQ ID 6: T877A_LNA and
- A DNA polynucleotide sequence or fragments thereof is a natural circulating tumor DNA.

Embodiments and preferred features can be combined together and are within the scope of the invention.

In a **second aspect,** the invention is directed to a LNA-blocker defined as a LNA-oligonucleotide sequence of 6 to 25 bases comprising a mixture of nucleic acid bases and LNA-monomer bases wherein the LNA-oligonucleotide sequence is complementing/hybridizing a specific DNA sequence of a DNA polynucleotide sequence encoding for a protein or fragments thereof or a DNA target gene wherein the specific DNA sequence is a Wild type DNA sequence for which one or more Mutations were identified and found related to none-response to a pharmaceutical treatment. In other words, the LNA-blocker is complementing a Wild type DNA sequence that is selected because of identified mutation(s) within this sequence. These Mutations are qualifying as a potential biomarker for identifying patient not-responding to a given pharmaceutical treatment.

Preferably, the LNA-oligonucleotide sequence is encoding for a protein wherein nucleotide mutations thereof results into dysfunction of the encoded protein.
More preferably, the LNA-oligonucleotide sequence is encoding for an Androgen Receptor (AR) or fragments thereof wherein nucleotide mutations thereof results into patient suffering from castration-resistant prostate cancer (CRPC).

Preferably, the Wild type DNA sequence is defined as such that one Mutation was identified within this sequence and found related to none-response to a pharmaceutical treatment. More preferably, the Wild type DNA sequence is defined as such that one Mutation was identified within this sequence and found related to none-response to a pharmaceutical treatment thereof due to dysfunctionment of the encoded protein. Even more preferably, the Wild type DNA sequence is defined as such that one Mutation was identified within this sequence and found related to none-response to a pharmaceutical treatment thereof due to dysfunctionment of the encoded protein and resulting into castration-resistant prostate cancer (CRPC) wherein the pharmaceutical treatment consists into administration of Flutamide or Bicalutamide to a patient in need.

Preferably, the Mutation is defined as above.

Preferably, the LNA-blocker is defined as a LNA-oligonucleotide sequence of 6, 7, 8, 9, 10, 11, 12, 13, 14, 15, 16, 17, 18, 19 or 20 bases comprising a mixture of nucleic acid bases and LNA-monomer bases. More preferably, the LNA-blocker is defined as a LNA-oligonucleotide sequence of bases comprising a mixture of 10 to 18 or 11 to 17 nucleic acid bases and LNA-monomer bases. Even more preferably, the LNA-blocker is defined as a LNA-oligonucleotide sequence of 11 or 17 bases, independently from each other, comprising a mixture of nucleic acid bases and LNA-monomer bases. Even more preferably, the LNA-blocker is defined as a LNA-oligonucleotide sequence of 11 bases selected from SEQ ID 5: W741C_LNA.

Even more preferably, the LNA-blocker is defined as a LNA-oligonucleotide sequence of 17 bases selected from SEQ ID 6: T877A_LNA.

In **first embodiment,** the LNA-blocker comprises 1 to 12, 2 to 12 or 3 to 12 LNA-monomer bases with the proviso that the LNA-blocker comprises at least 1 nucleic acid base. Preferably, the LNA-blocker comprises 7 to 11 LNA-monomer bases. More preferably, the LNA-blocker comprises 8, 9 or 10 LNA-monomer bases.

Even more preferably, the LNA-blocker comprises 8 LNA-monomer bases and is corresponds to SEQ ID 5: W741C_LNA.

Even more preferably, the LNA-blocker comprises 10 LNA-monomer bases and is corresponds to SEQ ID 6: T877A_LNA.

In **second embodiment,** the LNA-blocker is defined as beginning [5'] and ending [3'] with a LNA-monomer base.

Preferably, the LNA-blocker is defined as a LNA-oligonucleotide sequence of 10 bases selected from SEQ ID 7: W741C_LNA_np.

Preferably, the LNA-blocker is defined as a LNA-oligonucleotide sequence of 16 bases selected from SEQ ID 8: T877A_LNA_np.

In third embodiment, the LNA-blocker is defined as having a LNA-monomer base positioned in the central portion of the LNA-oligonucleotide sequence. Preferably, the LNA-blocker is defined having a LNA-monomer base positioned in the central portion of the LNA-oligonucleotide sequence wherein said LNA-monomer base is at the position of the Mutation.

Central portion of the LNA-oligonucleotide sequence means that the LNA-monomer base complementing the Mutation to be detected is located in the middle +/- 2 nucleotide of the LNA-oligonucleotide sequence.

The LNA-monomer base at the position of the Mutation corresponds to the Wild type nucleic acid base.

More preferably, the LNA-blocker is defined as SEQ ID 5: W741C_LNA having a LNA-monomer base G*₆ at the Mutation position.

More preferably, the LNA-blocker is defined as SEQ ID 6: T877A_LNA having a LNA-monomer base A*₇ at the Mutation position.

In a **fourth embodiment,** the LNA-blocker is carrying a protecting group defined as a 3' Inverted Thymidine (3InvdT) at the 3' extremity.
Preferably, the LNA-blocker is SEQ ID 5: W741C_LNA comprising a 3lnvdT.
Preferably, the LNA-blocker is SEQ ID 6: T877A_LNA comprising a 3lnvdT.

In a **fifth embodiment,** the LNA-blocker is defined as
- the LNA-oligonucleotide sequence of 6 to 25 bases comprising a mixture of nucleic acid bases and LNA-monomer bases wherein the LNA-oligonucleotide sequence is complementing/hybridizing a DNA polynucleotide sequence at a specific DNA sequence of a DNA polynucleotide sequence encoding for a protein or fragments thereof or a DNA target gene wherein the specific DNA sequence is a Wild type DNA sequence for which one or more Mutations were identified and found related to none-response to a pharmaceutical treatment,
- the LNA-oligonucleotide sequence comprises 1 to 12, 2 to 12 or 3 to 12 LNA-monomer bases, the LNA-oligonucleotide sequence is beginning and ending with a LNA-monomer base and
- the LNA-blocker is carrying a protecting group defined as an 3' Inverted Thymidine (3InvdT).

Preferably, the LNA-blocker is SEQ ID 5: W741C_LNA of 11 bases complementing/hybridizing a DNA at the specific position of the codon 741 of AR wherein the LNA-blocker
comprises 8 LNA-monomer bases,
begins with T* LNA-monomer base,
ends G* LNA-monomer base and
carries a protecting group defined as an 3' Inverted Thymidine (3InvdT).

Preferably, the LNA-blocker is SEQ ID 6: T877A_LNA of 17 bases complementing/hybridizing a DNA at the specific position of the codon T877A of AR wherein the LNA-blocker
comprises 10 LNA-monomer bases,
begins with C* LNA-monomer base,
ends C* LNA-monomer base and
carries a protecting group defined as an 3' Inverted Thymidine (3InvdT).

Preferably, the LNA-blocker is SEQ ID 7: W741C_LNA_np.

Preferably, the LNA-blocker is SEQ ID 8: T877A_LNA_np.

| LNA-Blockers | |
|---|---|
| SEQ ID 5: W741C_LNA | T*-C*-C*-**T**-G*-G*-**A**-T*-G*-G*/3InvdT/ |
| | T*-C*-C*-**T**-G*-G*-**A**-T*-G*-G*/3InvdT/ |
| | with highlighted LNA-monomer position of the nucleotide Mutation |
| | T*₁-C*₂-C*₃-**T**₄-G*₅-G*₆-**A**₇-T*₈-G*₉-G*₁₀/3InvdT₁₁/ |
| | with nucleotide position numbering |
| SEQ ID 6: T877A_LNA | C*-A*-G*-**T-T**-C*-A*-C*-T*-**T-T-T**-G*-**A**-C*-C*/3InvdT/ |
| | C*-A*-G*-**T-T**-C*-A*-C*-T*-**T-T-T**-G*-**A**-C*-C*/3InvdT/ |
| | with highlighted LNA-monomer position of the nucleotide Mutation |
| | C*₁-A*₂-G*₃-**T**₄-**T**₅-C*₆-A*₇-C*₈-T*₉-**T**₁₀-**T**₁₁-**T**₁₂-G*₁₃-**A**₁₄-C*₁₅-C*₁₆/3InvdT₁₇/ |
| | with nucleotide position numbering |
| SEQ ID 7: W741C_LNA_np | T*-C*-C*-**T**-G*-G*-**A**-T*-G*-G* |
| SEQ ID 8: T877A_LNA_np | C*-A*-G*-**T-T**-C*-A*-C*-T*-**T-T-T**-G*-**A**-C*-C* |

| | |
|---|---|
| "3InvdT" means 3' Inverted Thymidine. "np" means non-protected. "A*" "G*", "C*" and "T*" stand for LNA-monomer base. A, G, C, and T stand for natural and synthetic nucleic acid base. | |

DNA stands for genomic DNA and fragments thereof.

LNA-monomers are described above and drawing 5.

Embodiments and preferred features can be combined together and are within the scope of the invention.

In a **third aspect,** the invention is directed to
- the upstream primer of SEQ ID 1 W741C or SEQ ID 3 T877A and
- the downstream primer of SEQ ID 2: AR5_REV_W741C or SEQ ID 4: AR5_REV_T877A.

Primers:

| | |
|---|---|
| SEQ ID 1: AR1_FOR_W741C | GACCAGATGGCTGTCATTCA |
| SEQ ID 2: AR5_REV_W741C | AAAGCCGCCTCATACTGGAT |
| SEQ ID 3: AR1_FOR_T877A | CCCTACAGATTGCGAGAGAG |
| SEQ ID 4: AR5_REV_T877A | CAAGGCACTGCAGAGGAGTA |

The location of the primer has an influence on blocker efficiency. The upstream primer is designed so that it's attached to the template strand just a few bases upstream of the LNA-blocker. The closeness of the blocker to the 3'-end of the upstream primer prevented any elongation of the upstream primer at an early stage. If the upstream primer were to be located further upstream of the blocker, , it would continue to be elongated until polymerization was stopped by the LNA-blocker. But the melting temperature of the elongated upstream primer then exceeds the melting temperature of the LNA-blocker, which detach from the upstream primer between the elongation and denaturation steps, and the upstream primer will then continue to be elongated until the end.

In a **fourth aspect,** the invention is directed to a kit for detecting the presence of at least one nucleotide Mutation in a DNA polynucleotide sequence encoding for a protein or fragments thereof isolated from patient plasma, wherein the kit comprises a LNA-blocker as described in the second aspect wherein the DNA polynucleotide sequence or fragments thereof is a natural circulating tumor DNA fragments isolated from patient plasma.
LNA-blocker definition as in second aspect is here enclosed.

Preferably, the kit for detecting the presence of at least one nucleotide Mutation in a DNA polynucleotide sequence encoding for an Androgen Receptor (AR) or fragments thereof isolated from patient plasma, wherein the kit comprises a LNA-blocker defined as SEQ ID 5: W741C_LNA, SEQ ID 6: T877A_LNA, SEQ ID 7: W741C_LNA np or SEQ ID 8: T877A_LNA_np.

In a first embodiment, the kit comprises a LNA-blocker and an upstream primer wherein the upstream primer and the LNA-blocker are closely annealing onto the DNA fragments isolated from patient plasma.

Preferably, the kit comprises a LNA-blocker and an upstream primer wherein the upstream primer and the LNA-blocker are closely annealing onto the DNA fragments isolated from patient plasma and the LNA-blocker is defined as SEQ ID 5: W741C_LNA, SEQ ID 6: T877A_LNA, SEQ ID 7: W741C_LNA_np or SEQ ID 8: T877A_LNA_np and the upstream primer is defined as SEQ ID 1 W741C or SEQ ID 3 T877A.

In a second embodiment, the kit comprises a LNA-blocker, an upstream primer and a downstream primer wherein the upstream primer and the LNA-blocker are closely annealing onto the DNA fragments isolated from patient plasma.

Preferably, the kit comprises a LNA-blocker, an upstream primer and a downstream primer wherein the upstream primer and the LNA-blocker are closely annealing onto the DNA fragments isolated from patient plasma and the LNA-blocker is defined as SEQ ID 5: W741C_LNA, SEQ ID 6: T877A_LNA, SEQ ID 7: W741C_LNA_np or SEQ ID 8: T877A_LNA_np, the upstream primer is defined as SEQ ID 1 W741C or SEQ ID 3 T877A and the downstream primer of SEQ ID 2: AR5_REV_W741C or SEQ ID 4: AR5_REV_T877A.

Preferably, closely means that the distance between the last nucleic acid base of the upstream primer and the first nucleic acid base of the LNA-blocker is corresponding to a sequence of 3 to 10 nucleic acid bases of the DNA isolated from patient plasma.

The last nucleic acid base of the upstream primer means the 3' nucleic acid base of the upstream primer.

The first nucleic acid base of the LNA-blocker means the 5' nucleic acid base of the LNA-blocker.

For clarification purpose, the sequence of 3 to 10 nucleic acid bases of the DNA between the last nucleic acid base of the upstream primer and the first nucleic acid base of the LNA-blocker is not complemented by the upstream primer or the LNA-blocker.

More preferably, closely means that the distance between the last nucleic acid base of the upstream primer and the first nucleic acid base of the LNA-blocker is corresponding to a sequence of 3 to 7 or 4 to 6 nucleic acid bases of the DNA isolated from patient plasma, even more preferably 4 or 6.

Preferably, the upstream primer is SEQ ID 1 W741C or SEQ ID 3 T877A.

Preferably, the downstream primer is SEQ ID 2: AR5_REV_W741C or SEQ ID 4: AR5_REV_T877A. Preferably, the LNA blocker is SEQ ID 5: W741C_LNA, SEQ ID 6: T877A_LNA, SEQ ID 7: W741C_LNA_np or SEQ ID 8: T877A_LNA_np.

Preferably, the DNA polynucleotide sequence is encoding for a protein wherein oligonucleotide mutations thereof results into dysfunction of the encoded protein leading to castration-resistant prostate cancer (CRPC).

More preferably, the DNA polynucleotide sequence is encoding for an Androgen Receptor (AR) or fragments thereof wherein oligonucleotide mutations thereof results into castration-resistant prostate cancer (CRPC) due to administration to a patient in need of Flutamide or Bicalutamide.

Embodiments and preferred features can be combined together and are within the scope of the invention.

### Definitions:

PCR stands for polymerase chain reaction.

LNA clamp qRT-PCR stands for Locked Nucleic Acids clamp quantitative Real Time-PCR. Such PCR is well known in the art and is conducted on standard real time PCR device. Primers are ordered from a primer provider. The LNA blockers are marketed by the company EXIQON in Denmark.

PCR amplification is detected using a SYBR-Green moiety as provided by Qiagen in the QuantiTect-SYBR-Green PCR kit.

PCR Primers are used in standard chemical synthesis methods or are produced using enzymatic methods or any other known method. Such methods can range from standard enzymatic digestion followed by nucleotide fragment isolation (see for example, Sambrook et al., Molecular Cloning: A Laboratory Manual, 2nd Edition (Cold Spring Harbor Laboratory Press, Cold Spring Harbor, N.Y., 1989) Chapters 5, 6, incorporated herein by reference) to purely synthetic methods, for example, by the cyanoethyl phosphoramidite method using a Milligen or Beckman System 1Plus DNA synthesizer (for example, Model 8700 automated synthesizer of Milligen-Biosearch, Burlington, Mass. or ABI Model 380B). Synthetic methods useful for making oligonucleotides are also described by Ikuta et al., Ann Rev. Biochem. 53:323-356 (1984), (phosphotriester and phosphite-triester methods, incorporated herein by reference), and Narang et al., Methods Enzymol., 65:610-620 (1980), incorporated herein by reference, (phosphotriester method). Protein nucleic acid molecules can be made using known methods such as those described by Nielsen et al., Bioconjug. Chem. 5:3-7 (1994), incorporated herein by reference. "Primers" are a subset of probes which are capable of supporting some type of enzymatic manipulation and which can hybridize with a target nucleic acid such that the enzymatic manipulation can occur. A primer can be made from any combination of nucleotides or nucleotide derivatives or analogs available in the art which do not interfere with the enzymatic manipulation. A nucleotide is a molecule that contains a base moiety, a sugar moiety and a phosphate moiety. Nucleotides can be linked together through their phosphate moieties and sugar moieties creating an internucleoside linkage. The base moiety of a nucleotide can be adenin-9-yl (A), cytosin-1-yl (C), guanin-9-yl (G), uracil-1-yl (U), and thymin-1-yl (T). The sugar moiety of a nucleotide is a ribose or a deoxyribose. The phosphate moiety of a nucleotide is pentavalent phosphate. A non-limiting example of a nucleotide would be 3'-AMP (3'-adenosine monophosphate) or 5'-GMP (5'-guanosine monophosphate). The term "nucleotide" includes nucleotides and nucleotide analogs, preferably groups of nucleotides comprising oligonucleotides, and refers to any compound containing a heterocyclic compound bound to a phosphorylated sugar by an N-glycosyl link or any monomer capable of complementary base pairing or any polymer capable of hybridizing to an oligonucleotide. The term "oligonucleotide" means a naturally occurring or synthetic polymer of nucleotides, preferably a polymer comprising at least three nucleotides and more preferably a polymer capable of hybridization. Oligonucleotides may be single-stranded, double-stranded, partially single-stranded or partially double-stranded ribonucleic or deoxyribonucleic acids, including selected nucleic acid sequences, heteroduplexes, chimeric and hybridized nucleotides and oligonucleotides conjugated to one or more nonoligonucleotide molecules. In general, the nucleotides comprising a oligonucleotide are naturally occurring deoxyribonucleotides, such as adenine, cytosine, guanine or thymine linked to 2'-deoxyribose, or ribonucleotides such as adenine, cytosine, guanine or uracil linked to ribose. However, an oligonucleotide also can contain nucleotide analogs, including non-naturally occurring synthetic nucleotides or modified naturally occurring nucleotides. Such nucleotide analogs are well known in the art and commercially available, as are polynucleotides containing such nucleotide analogs (Lin et al., Nucl. Acids Res. 22:5220-5234 (1994); Jellinek et al., Biochemistry 34:11363-11372 (1995); Pagratis et al., Nature Biotechnol. 15:68-73 (1997), each of which is incorporated herein by reference).

A polynucleotide comprising naturally occurring nucleotides and phosphodiester bonds can be chemically synthesized or can be produced using recombinant DNA methods, using an appropriate polynucleotide as a template. In comparison, a polynucleotide comprising nucleotide analogs or covalent bonds other than phosphodiester bonds generally will be chemically synthesized, although an enzyme such as T7 polymerase can incorporate certain types of nucleotide analogs into a polynucleotide and, therefore, can be used to produce such a polynucleotide recombinantly from an appropriate template (Jellinek et al., supra, 1995, incorporated herein by reference). The term "primer pair" refers to a set of primers Including a 5' "upstream primer" or "forward primer" that hybridizes with the complement of the 5' end of the DNA sequence to be amplified and a 3' "downstream primer" or "reverse primer" that hybridizes with the 3' end of the sequence to be amplified. As will be recognized by those of skill in the art, the terms "upstream" and "downstream" or "forward" and "reverse" are not intended to be limiting, but rather provide ii!ustrative orientation in particular embodiments.

### gDNA means genomic DNA.

The androgen receptor (steroid receptor) is divided structurally into four domains: an N-terminal region with a transactivation function, a DNA-binding domain with a nuclear localization signal (NLS) and a ligand-binding domain (LBD) [95]. The AR acts as a ligand-dependent transcription factor. It is located in the cytoplasm in the absence of testosterone and forms a complex with heat shock proteins. If testosterone or its metabolite dihydrotestosterone (DHT) binds to the AR, a change in conformation occurs which leads to dissociation of the heat shock proteins. The NLS can then be detected by import proteins and the AR is translocated into the nucleus, where, as a homodimer, it binds to the androgen response element (ARE) upstream of the target genes [95-97].
A publication by Watson and Sawyers (2009) included the hypothesis of a mutation in the AR ligand-binding domain leading to a gain of function in the AR as a result of the use of alternative steroid ligands [94]. Binding to alternative ligands is suggested by the altered structure of the binding pocket [98]. In 1990, Verlscholte *et al.* discovered a missense mutation in the ligand-binding domain of the AR in the LNCaP human prostate cancer cell line (T877A in exon H of the human AR) [99]. This mutation resulted in an increase in the range of ligands which led to AR activation. In addition to glucocorticosteroids [100], substances such as estrogens and hydroxyflutamide also led to transactivation [99, 101]. The T877A mutation is located in the AF-2 domain of exon H. AF-2 binds coregulatory proteins and interacts with the transactivation domain. The T877A missense mutation has also been detected in some cases of metastatic prostate cancer treated with hydroxyflutamide [102]. Bicalutamide, on the other hand, did not activate the mutated AR in LNCaP and continued to act as an antagonist [101, 103]. An AR mutation has also been detected in patients under Bicalutamide therapy [104]. Yoshida *et al.* (2005) received the KUCaP cell line containing a W741C mutation (exon E of the human AR) from a liver metastasis of prostate cancer in a patient [105]. As a result of this W741C mutation, Bicalutamide exerts an agonist effect on the AR. Flutamide continues to act as an antagonist.

### Experimental part

### A. Method:

| Table 2.3: Kits used | |
|---|---|
| Kit | Manufacturer |
| Agilent_DNA_1000_Kit | Agilent_Technologies |
| MinElute_PCR_Purifiction_Kit | QIAGEN |
| QIAamp^{®} DNA_Mini_and_Blood_Mini_kit | QIAGEN |
| QIAamp^{®} Circulating_Nucleic_Acid_Kit | QIAGEN |
| QuantiTect_SYBR_Green_PCR_+_UNG_Kit | QIAGEN |

### Oligonucleotides

The genomic sequence of the human androgen receptor comes from the NG_009014 RefSeq (Reference Sequence) database sequence of the National Center for Biotechnology Information (NCBI). The mRNA sequence of the human androgen receptor was taken from the NG_000044 RefSeqGene sequence. The primers were ordered from TIB MOLBIOL GmbH (Berlin, Germany) and purified by means of HPLC. The two primer pairs AR1_FOR_W741C and AR5_REV_W741C and the primer pairs AR1_FOR_T877A and AR5_REV_T877A amplify the genomic DNA region in which the base substitutions (T877A, W741C) in the human AR sequence are located.

The locked nucleic acid (LNA) probes were designed in-house and ordered from the company EXIQON in Denmark. In order to prevent the DNA polymerase-induced elongation, an inverted thymine (3InvdT) was added to the 3' of the LNA probe. The LNA probes consisted of a mixture of normal bases and specific LNA monomers, which are indicated by a '*' in Table 2.5. The T877A LNA clamp is complementary to a 16 nt region (genomic DNA (gDNA) region: 184673 bp - 184688 bp) which covers codon 877 of the human AR. The complementary region which covers codon 877 is highlighted in bold. The W741C LNA clamp is complementary to a 10 nt region (gDNA region: 178494 bp - 178503 bp) which covers codon 741. The complementary region which covers codon 741 is highlighted in bold. Both LNA clamps were designed using EXIQON's LNA Oligo Tools LNA Oligo Tm Prediction and LNA Oligo Optimizer.

| Table 2.5: Oligonucleotides and amplicons | | | |
|---|---|---|---|
| Primers | Sequence [5'-3'] | Tm | Amplicon/size |
| AR1_FOR_W741C | GACCAGATGGCTGTCATTCA | 60 °C | AR_W741C (279 bp in Exon E) |
| AR5_REV_W741C | AAAGCCGCCTCATACTGGAT | 61 °C | |
| AR1_FOR_T877A | CCCTACAGATTGCGAGAGAG | 58 °C | AR_T877A (259 bp in Exon H) |
| AR5_REV_T877A | CAAGGCACTGCAGAGGAGTA | 59°C | |
| LNA-blockers | | | |
| W741C_LNA | T*-C*-C*-T-G*-G*-A-T*-G*-G*/3InvdT/ | 76 °C | |
| T877A_LNA | C*-A*-G*-T-T-C*-A*-C*-T*-T-T-T-G*-A-C*-C*/3InvdT/ | 74 °C | |

### Plasma preparation

In order to obtain 4 ml plasma per patient, venous blood was collected into EDTA plasma Monovettes^{®} (Sarstedt) in the hospital and centrifuged for 10 min at 1,900 x g and 4 °C using a swing-out rotor. The supernatant (plasma) was then divided into aliquots, placed in intermediate storage at a temperature of -20 °C and transported to Bayer Pharma AG, where it was stored at -80 °C. For subsequent tests, these frozen plasma samples were thawed at RT and centrifuged for 10 min at 16,000 x g and 4 °C in order to remove additional cell debris and contamination of circulating nucleic acids by gDNA and RNA of dead blood cells. As a control, 1 ml plasma was obtained from each of 6 healthy blood donors by Clinical Pharmacology, Bayer Pharma AG, treated in the same way and prepared for free-circulating DNA isolation.

### isolation of free-circulating DNA

Free, circulating DNA (cfDNA) was isolated with the QIAGEN QIAamp^{®} Circulating Nucleic Acid Kit using a vacuum (QIAGEN QIAvac24 Plus^{®} vacuum pump) in accordance with the manufacturer's instructions. A carrier RNA (1 µg) was added to increase binding of the rare nucleic acids to the silica membrane. Because of the low yield of cfDNA, the RNA carrier used and the buffer components, quantification by means of UV absorption spectrometry was not possible. The cfDNA content was determined by means of qRT-PCR. A fixed pipetting volume of 5 µl was used for this, see 2.4.3. The minimal volume of 1 ml plasma per patient/subject was used for the assay. Elution was performed using buffer AVE in a minimal volume of 25 µl (approximately 20 µl eluate).

### LNA clamp quantitative real-time PCR

For the quantitative real-time PCR (qRT-PCR), the QuantiTect SYBR Green PCR + UNG Kit and the 7500 Fast Real-Time PCR Cycler (Applied Biosystems) were used in accordance with the manufacturer's instructions. The qRT-PCR was analyzed using the ABI 7500 v.2.0.5 software package. In order to prevent contamination from any previous PCR, uracil N-glycosylase was added in accordance with the manufacturer's instructions. Both of the following were used initially for the qualitative analysis: a melting curve run after the qRT-PCR in accordance with the manufacturer's instructions and an analysis of the PCR products by means of gel electrophoresis. The optimal annealing temperatures were determined in advance by means of conventional PCR with a temperature gradient. The LNA clamp qRT-PCR was developed to detect gDNA displaying missense mutations (T877A & W741C) in the human AR. In order to determine the ratio of mutated to WT-blocked AR amplification, each sample (template) was amplified in two independent PCR reactions with and without LNA probe which is 100% complementary to WT. The data were analyzed using a modified delta-delta Ct method (Ct or cycle threshold: cycle in which the fluorescence rises significantly above the background fluorescence and the amplification is in the exponential phase). The delta Ct value was determined from the difference between the Ct values of the sample with LNAs (WT is inhibited) and without LNAs (WT is not inhibited). This was done both for the WT sample (no point mutation in the AR reference sample) and for the sample to be analyzed (pure mutant, mutant in WT background, patient sample). The delta-delta Ct was determined from the difference between the delta Ct value of the WT sample and the delta Ct value of the sample to be analyzed. This value reflects the efficiency of the blocker [86, 120].
(I) Delta Ct (WT) = Ct (WT with LNAs) - Ct (WT without LNAs).
(II) Delta Ct (mutant) = Ct (mutant with LNAs) - Ct (mutant without LNAs).
(I+II) Delta-delta Ct (blocker efficiency) = delta Ct (WT) - delta Ct (mutant).

All the cell culture samples were determined in triplicate and repeated in three different runs. Because it was not possible to determine the DNA concentration of the clinical samples, measurements were performed on each 5 µl as duplicates with and without LNA blocker. All the LNA qRT-PCRs were pipetted under sterile conditions under a laminar flow hood in order to prevent contamination with gDNA. A negative control (water instead of template) was run at the same time.

| Table 2.14: PCR cycle of the W741C AR amplicon | | | |
|---|---|---|---|
| Step | Time [s] | Temp. [°C] | Number of cycles |
| 1._Aktivation_UNG_ | 120 | 50 | 1 |
| 2._Initiale_Denaturation_ | 15 | 95 | 1 |
| 3._Denaturation_ | 15 | 94 | |
| 4._Annealing_ | 30 | 55 | 3.-5.,_50_Wdh._ |
| 5._Elongation | 30 | 74 | |

It should be noted that, during the PCR cycle of the T877A AR amplicon, the annealing temperature had to be 60 °C instead of 55 C, and the elongation temp. 72 C instead of 74 C.

### Sequencing

In order to verify the point mutations in the human AR (T877A & W741C), the LNA clamp qRT-PCR products (pool of the duplicates or triplicates) were analyzed by means of sequencing. The sequencing was carried out by the company Services in Molecular Biology (SMB) in the Institute of Biology at Humboldt University, Berlin, using the chain-termination method [121]. 50 ng DNA (259 bp and 279 bp amplicons) in 5 µL RNase-free water were used per sequencing batch. The AR5_T877A_REV and AR5_W741C_REV primers were used for the sequencing.

### B. Results

### 1. LNA blocker and primer design

As a first step, the LNA blockers were designed for both mutations, along with the primers for amplification. With regard to the design of the LNA blockers, it was important for these to be located in a central position over the base substitution to be analyzed. They are made up of a mixture of deoxynucleotides and LNA monomers. A probe consisting of LNA monomers alone would have increased the self-complementarity of the probe, on the other hand. This would have also resulted in excessive inhibition of the gDNA of the mutation. A duplet or triplet of LNA monomers was placed directly over the codon to be analyzed in order to ensure the greatest possible discrimination between inhibited WT and amplified mutation. LNA monomers were added to both LNA blockers at the 5'- and 3'-end in order to protect them from the exonuclease function of the DNA polymerase. The LNA blockers were designed so that the Tm of the perfect match (WT + blocker) was higher than the elongation temperature. The Tm of the mismatch (mutation + blocker) needed to be lower than the elongation temperature. The purpose of this was to inhibit amplification of the WT template during elongation. Amplification of the mutation needed to be made possible, on the other hand. The Tm of the blockers was initially established on the basis of the standard elongation temperature of 72 °C. With regard to the primer design, it was important for the primers to be located upstream and downstream of the point mutations to be analyzed (see Drawing 8 and Drawing 7).Both forward primers were designed so that they attached to the template strand just a few bases upstream of the blocker. The closeness of the blocker to the 3'-end of the forward primer prevented any elongation of the forward primer at an early stage, which resulted in an increase in blocker efficiency. A 259 bp (T877A) and 279 bp (W741C) PCR fragment was amplified (see Drawing 7 and Drawing 8, Drawing 9 and Drawing 10).

### 2. Optimization of the LNA clamp qRT-PCR

### 2.1 Optimization of the annealing and elongation temperature

First, the annealing temperature was determined empirically by means of conventional PCR. For both assays, a temperature gradient of 53-63°C was run in 1°C steps. Optimal annealing temperatures were established of 55 C for the W741C assay and 60 °C for the T877A assay (data not shown). The optimal elongation temperature was also determined empirically. Temperatures of 72-76°C were tested in 1°C steps for both assays. A temperature of 74°C was selected for the W741C assay and 72 °C for the T877A assay. The elongation temperatures for both assays were 2 C below the specific Tm values of the LNA blockers (Tm of T877A-LNA: 74°C; Tm of W741C-LNA: 76 C). This ensured that the blocker remained bound firmly to the WT during elongation. Although an elongation temperature lower than 72 C/74 C would have resulted in even greater inhibition of WT, there would also then have been a risk of mutant inhibition. Both fragments were amplified with the aid of the primers without the formation of non-specific fragments. This was verified by means of subsequent melting curve and gel analysis (data not shown). The non-template control (NTC; H2O instead of DNA) included in each run and the control for LNA blocker specificity (primer for amplifying the W741C amplicon and T877A blocker, vice versa) were both negative (data not shown).

### 2.2 Optimization of the concentration of the gDNA and of the LNA blocker

In order to optimize the concentration of gDNA, the prostate cancer cell line LAP-C4 was used as a model for the wild type, LNCaP as a model for the T877A mutant and KUCaP-1 as a model for the W741C mutant. In order to establish the optimal gDNA concentration, 5 ng, 25 ng and 50 ng gDNA were used in the T877A assay (data not shown). The LNA concentration was first titrated roughly in the range of 1-10 µM. After no increase in blocker efficiency was detected in the T877A assay above a concentration of 1 µM, a fine titration was performed in the range of 0.1-1 µM. There was an increase in blocker efficiency up to an LNA concentration of 0.5 µM, and it then remained constant with additional increases in the LNA concentration (see Drawing 11 and Table 3.15). Peak blocker efficiency in respect of the gDNA was reached at 25 ng. Despite optimization, slight inhibition of the gDNA of the mutation was detectable (see Drawing 11). For the T877A batch, 25 ng gDNA and a final LNA concentration of 0.5 µM were established for subsequent tests.

Drawing 11 shows the optimization of the T877A batch. LNAs were used in final concentrations of 0.1-1 µM and 25 ng gDNA. The delta Ct value relating to the final LNA concentration in µM was applied. The delta Ct values of the WT (LAP-C4) are represented in black and those of the mutation (LNCaP) in white. All the tests were performed in technical replicates (n=3). The delta-delta Ct value has been represented as an arrow and is a measure of blocker efficiency. The Ct values of the WT and of the mutation were determined in the presence and absence of the LNA blocker. The difference in the Ct values yielded the delta Ct. Blocker efficiency was expressed in the form of the delta-delta Ct value and calculated as the difference between the delta Ct values for WT and mutation. An optimal delta-delta Ct value results from a high delta Ct value for the WT (strong inhibition) and a delta Ct value as low as possible (little/no inhibition) for the mutation. The parameters inside a gray border were used for all other experiments involving the T877A batch. The error bars indicate the standard deviation.

| Table 3.15: Delta-delta Ct values of the T877A-LNA batch | |
|---|---|
| **c(T877A-LNA)** | **Delta-delta Ct** |
| 0,1_µM | 4,3_ |
| 0,2_µM | 9,1 |
| 0,3_µM_ | 10,7_ |
| 0,4_µM_ | 11,3_ |
| 0,5_µM_ | 11,6_ |
| 1,0_µM_ | 11,2_ |

No titration of the gDNA was performed for the W741C-LNA batch. The optimal total DNA of 25 ng was used on the basis of the titration results of the preceding T877A-LNA batch. Because the T877A LNA blocker displayed its greatest effectiveness as an inhibitor at a final concentration of 0.5 µM, titration was performed from a final concentration of 0.5-5 µM in the W741C-LNA batch. There was an increase in blocker efficiency up to an LNA concentration of 3 µM, and it then remained constant with additional increases in the LNA concentration (see Drawing 12 and Table 3.16). Despite optimization, slight inhibition of the gDNA of the mutation was detectable (see Drawing 12). Because inhibition of the WT DNA was less effective than that of the WT DNA in the T877A-LNA batch even in the higher concentration range (0.5-5 µM), an attempt was made to increase the efficiency of the PCR by titration of MgCl2. One of the effects of MgCl2, as a cofactor of the DNA polymerase, is to increase the efficiency of the PCR. The highest LNA blocker efficiency was reached at a final MgCl2 concentration of 4 mM (standard: 2.5 mM). At a final LNA concentration of 3 µM, the delta-delta Ct was increased by MgCl2 from approximately 8 to approximately 11 (data not shown). For the W741C batch, 25 ng gDNA and a final LNA concentration of 3 µM were established for subsequent tests.

Drawing 12 shows the optimization of the LNA concentration of the W741C batch. For optimization of the W741C batch, LNAs were used in final concentrations of 0.5-5 µM and 25 ng gDNA. The delta Ct value relating to the final LNA concentration in µM was applied. The delta Ct values of the WT (LAP-C4) are represented in black and those of the mutation (KUCaP) in white. All the tests were performed in technical replicates (n=3). The delta-delta Ct value has been represented as an arrow and is a measure of blocker efficiency. The Ct values of the WT and of the mutation were determined in the presence and absence of the LNA blocker. The difference in the Ct values yielded the delta Ct. Blocker efficiency was expressed in the form of the delta-delta Ct value and calculated as the difference between the delta Ct values for WT and mutation. An optimal delta-delta Ct value results from a high delta Ct value for the WT (strong inhibition) and a delta Ct value as low as possible (little/no inhibition) for the mutation. MgCl2 was added in a final concentration of 4 mM to improve efficiency. The parameters inside a gray border were used for all other experiments involving the W741C batch. The error bars indicate the standard deviation.

| Table 3.16: Delta-delta Ct values of the W741C-LNA batch | |
|---|---|
| **c(W741C-LNA)** | **Delta-delta Ct** |
| 0,5_µM | 2,9 |
| 1,0_µM | 5,0 |
| 2,0_µM_ | 10,3 |
| 3,0_µM_ | 11,0 |
| 4,0_µM_ | 11,0 |
| 5,0_µM_ | 11,2 |

### 2.3: Use of standard dilutions to determine the detection limit

In order to determine the detection limit at which the previously established LNA assays were capable of detecting mutated tumor DNA in the presence of an excess of WT, standard dilution series in the range of 1:10-1:10,000 (mutant DNA in WT DNA) were prepared. It was found that the more mutated DNA a sample contained, the smaller the differences were between amplification with and without LNA blocker. The greater the WT background was in each batch, the weaker was the discrimination between the dilution and the WT with LNAs (see Drawing 13, top and bottom diagrams). In the T877A assay, a theoretical detection limit of 4,000-8,000 (2 ^ delta-delta Ct = 2¹²-2¹³) was determined in advance on the basis of a maximum delta-delta Ct of approximately 12-13 (calculated from pure WT and mutant DNA) (see Table 3.15 and Table 3.17). The detection limit was confirmed by the standard dilution series. At a dilution of 1:1,000, a clear delta-delta Ct of 2.8 was still detectable, whereas the 1:10,000 dilution was hardly distinguishable from the WT (delta-delta Ct of 1.3) (see Drawing 13, top diagram, Table 3.15 and Table 3.17). Detection of 1:10,000 (mutant DNA in WT DNA) could no longer be resolved. In the W741C assay, a theoretical detection limit of 1,000-2,000 (2 ^ delta-delta Ct = 2¹¹ - 2¹²) was determined in advance on the basis of a maximum delta-delta Ct of approximately 10-11 (calculated from pure WT and mutant DNA) (see Table 3.16 and Table 3.17). This detection limit was more or less confirmed by the standard dilution series. While the mutation was still discriminated weakly in the 1:1,000 dilution (delta-delta Ct of 1.0), the mutation was almost indistinguishable from the WT with LNA blocker at a dilution of 1:10,000. This means that the mutation could no longer be detected at 10,000 dilution in WT (see Drawing 13, bottom diagram, Table 3.16 and Table 3.17). In general, a fluctuation of +/- 1 in the delta-delta Ct value was detectable in the respective delta-delta Ct results (comp. Table 3.15 and Table 3.16 with Table 3.17). It should be borne in mind in connection with this that blocker efficiency is calculated from Ct values which are subject to assay-related fluctuations within a certain range. Blocker efficiency can therefore only be used in an approximate manner to estimate the achievable detection limit.

Drawing 13 shows the determination of the mutant DNA detection limit in the presence of an excess of WT DNA using standard dilutions. The quantity of gDNA used in each case was 25 ng. The delta Ct value relating to WT DNA, mutant DNA and dilutions of 1:10-1:10,000 (mutant DNA in WT DNA) was applied. The delta Ct value was calculated from the Ct value of the dilutions or of the WT (reference) with and without addition of LNAs. The delta-delta Ct value was obtained from the difference in the Ct value for the dilutions and for the WT (reference). The top diagram shows the dilution series for the T877A batch. The bottom diagram shows the dilution series for the W741C batch. All the tests were performed in technical replicates (n=3). The error bars indicate the standard deviation.

| Table 3.17: **Delta-delta** Ct values of the W741C and T877A standard dilutions | | |
|---|---|---|
| **Sample** | **Delta-delta Ct W741C-LNA 3 µM** | **Delta-delta Ct** |
| Mut | 10,3 | 13,0 |
| 1:10 | 6,6 | 9,2 |
| 1:100 | 3,2 | 5,2 |
| 1:1000 | 1,0 | 2,8 |
| 1:10000 | 0,3 | 1,3 |
| WT | 0,0 | 0,0 |

### 3: Sequence analysis for verifying detection limits

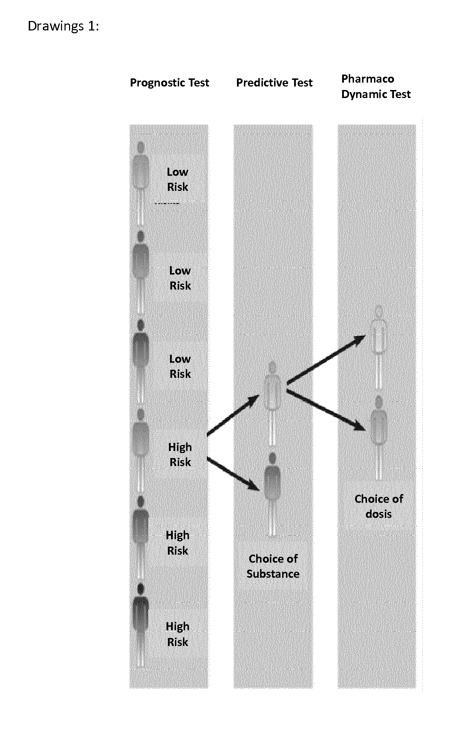

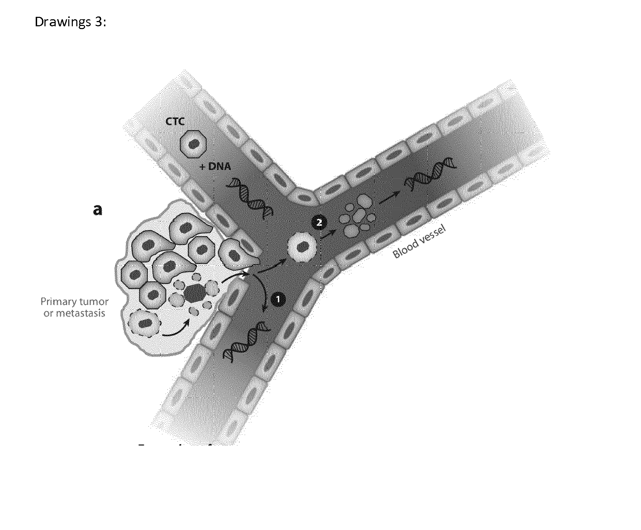

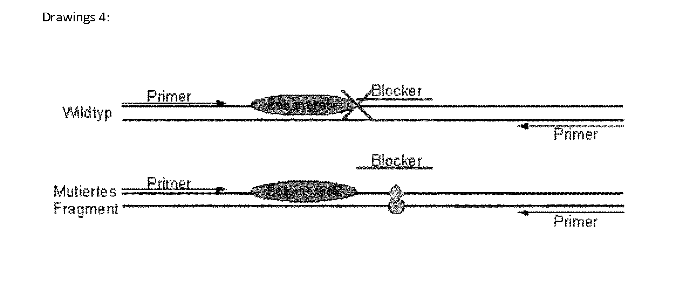

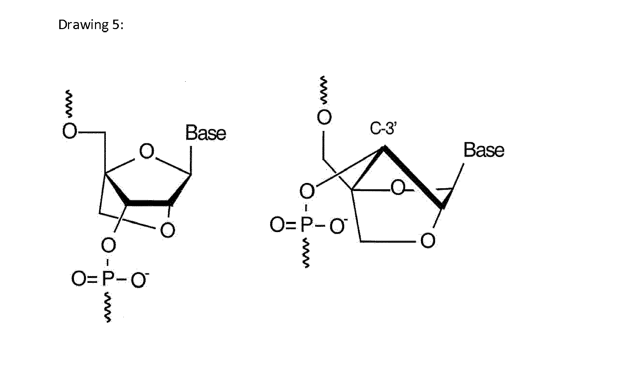

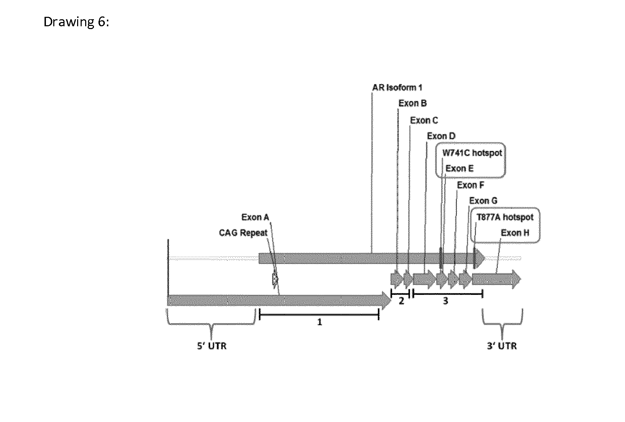

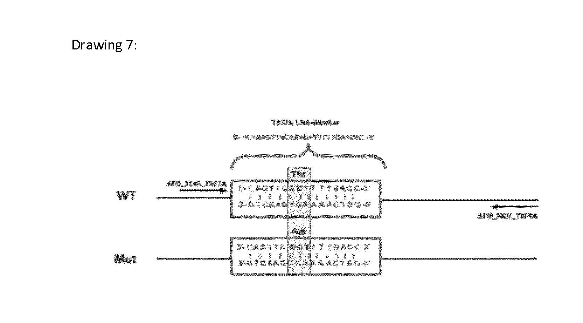

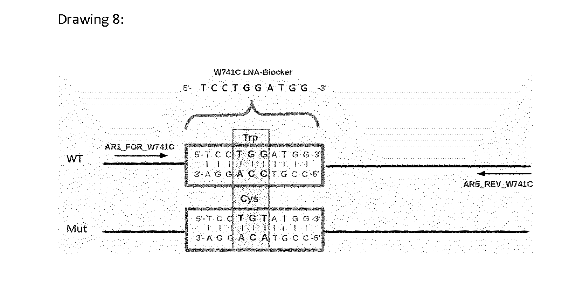

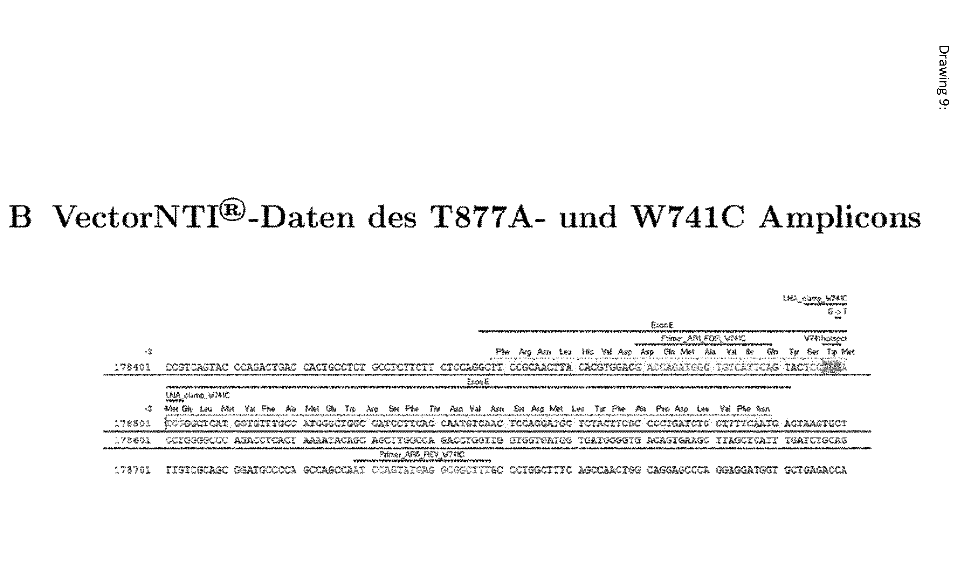

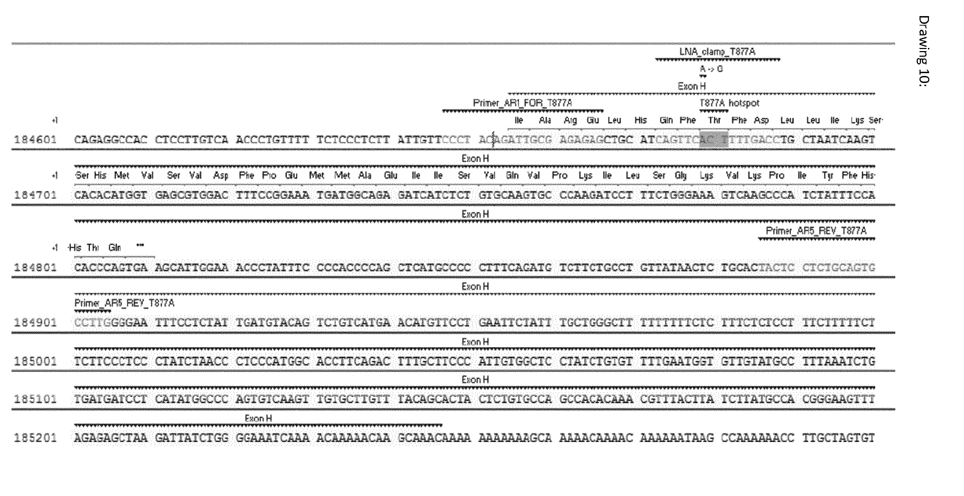

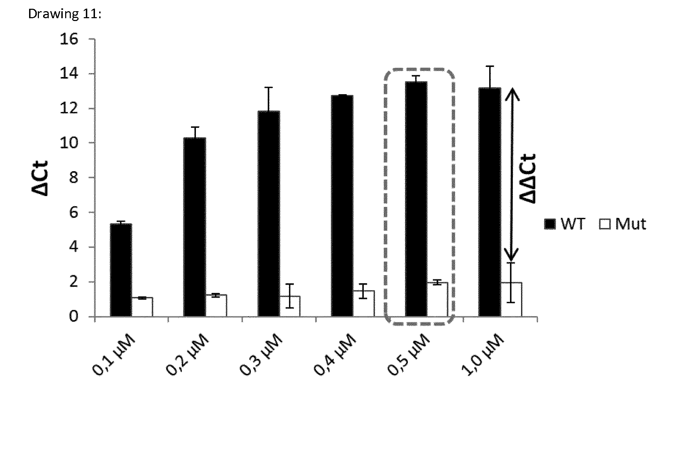

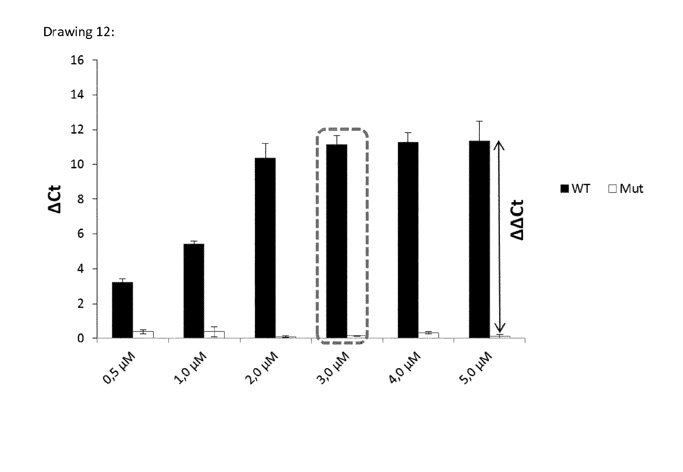

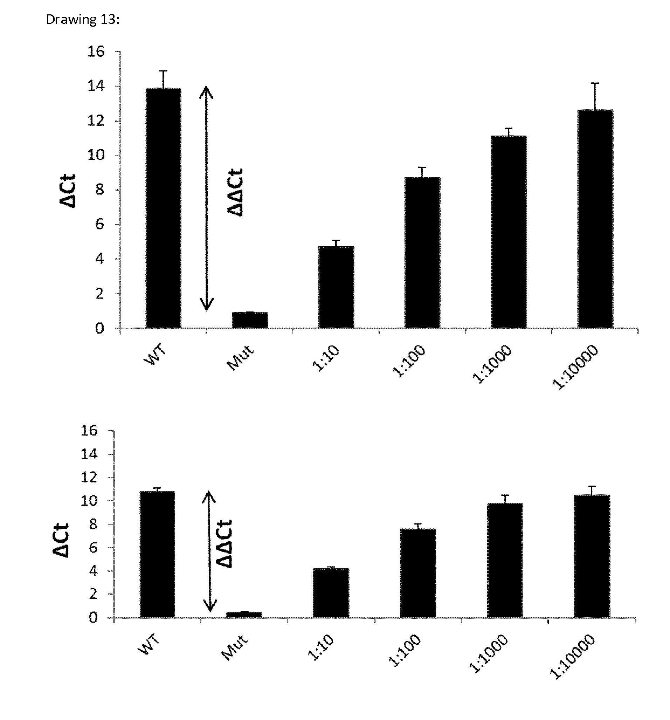

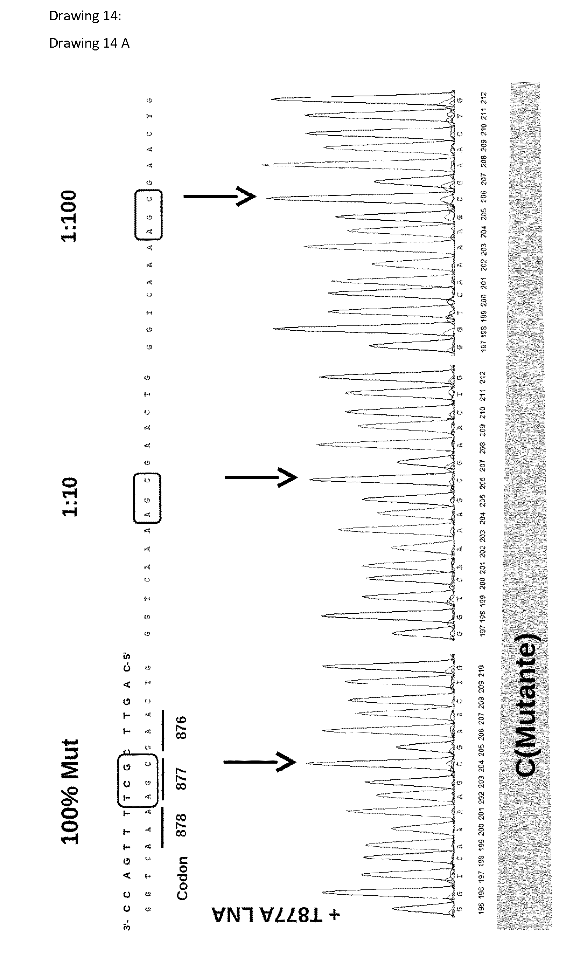

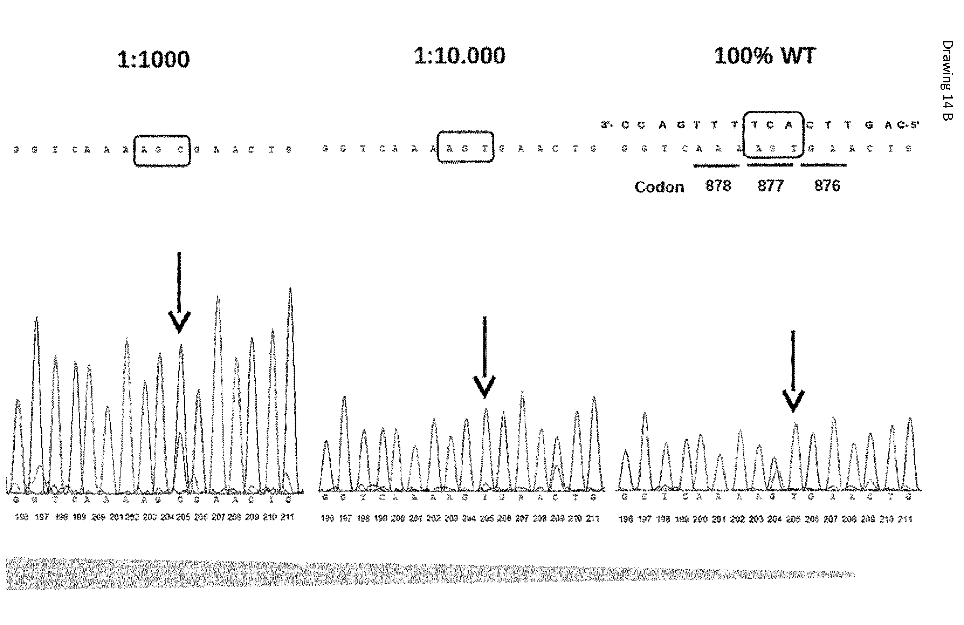

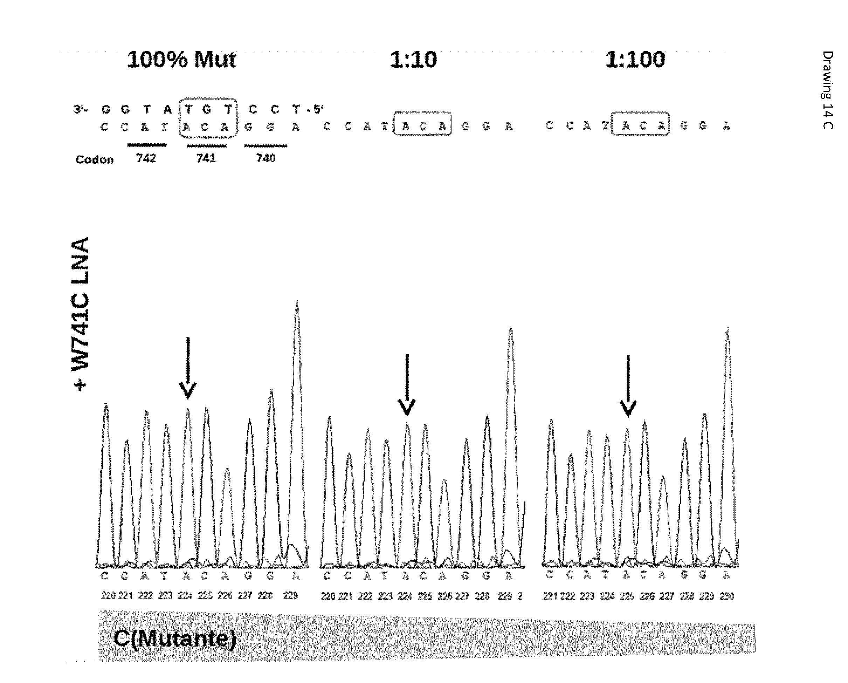

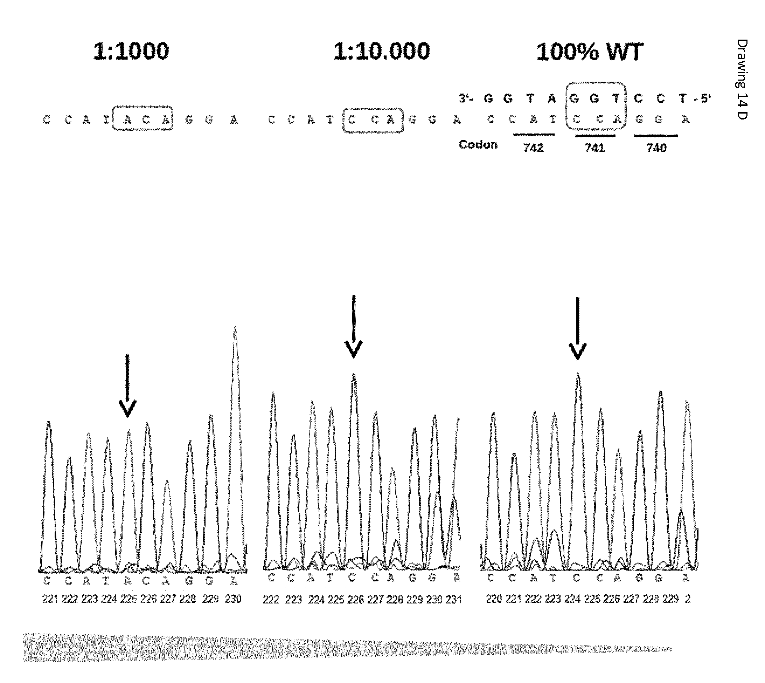

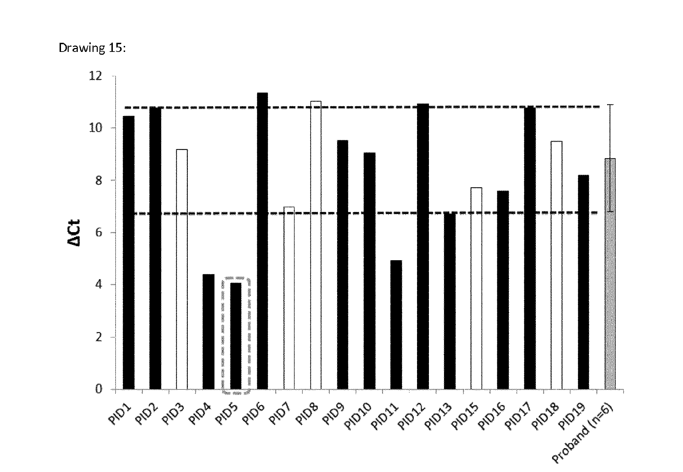

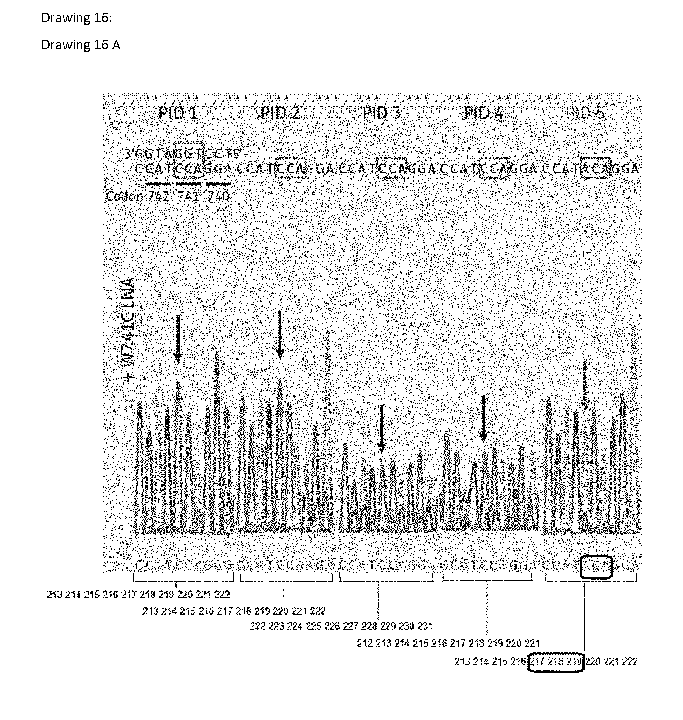

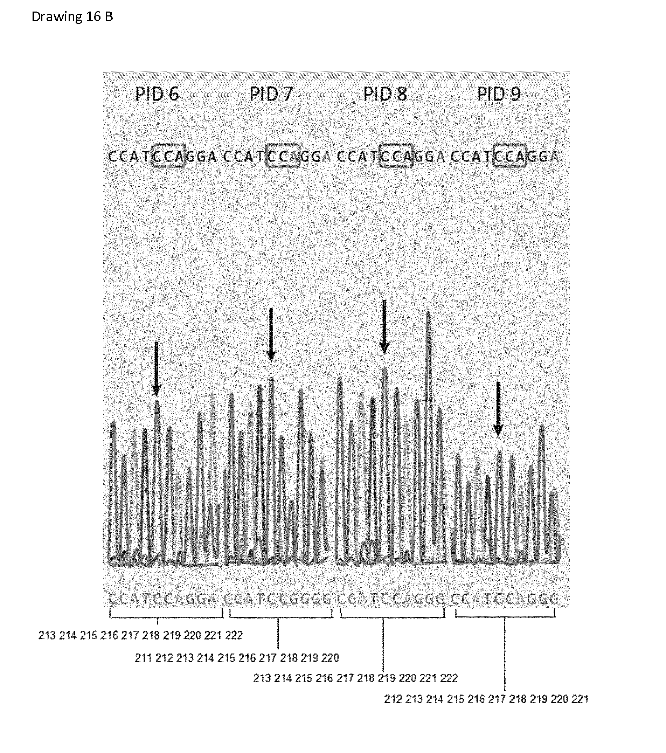

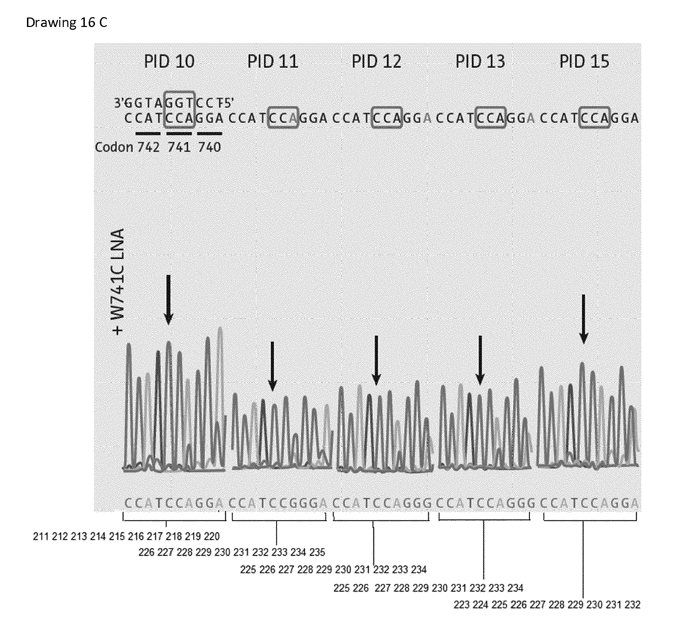

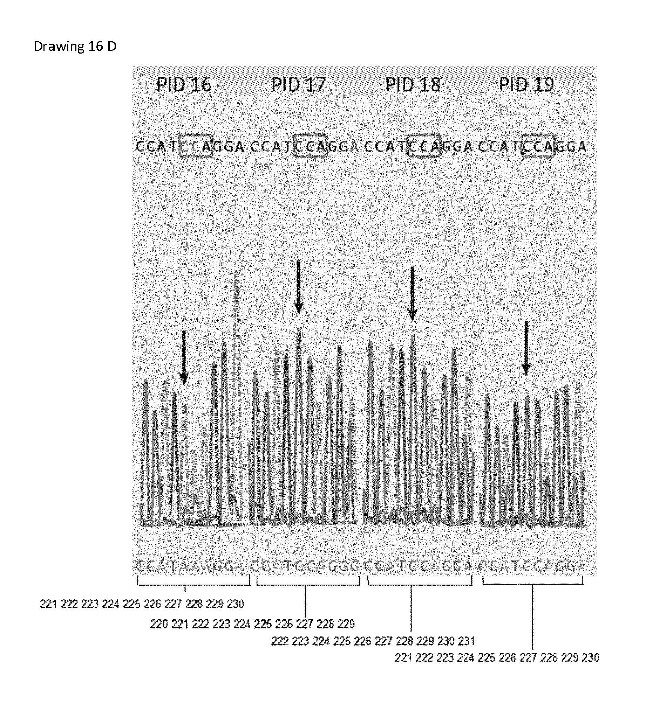

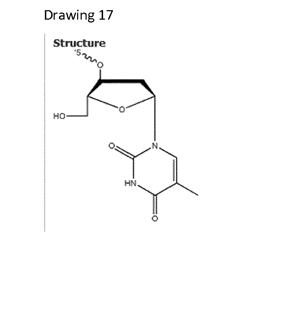

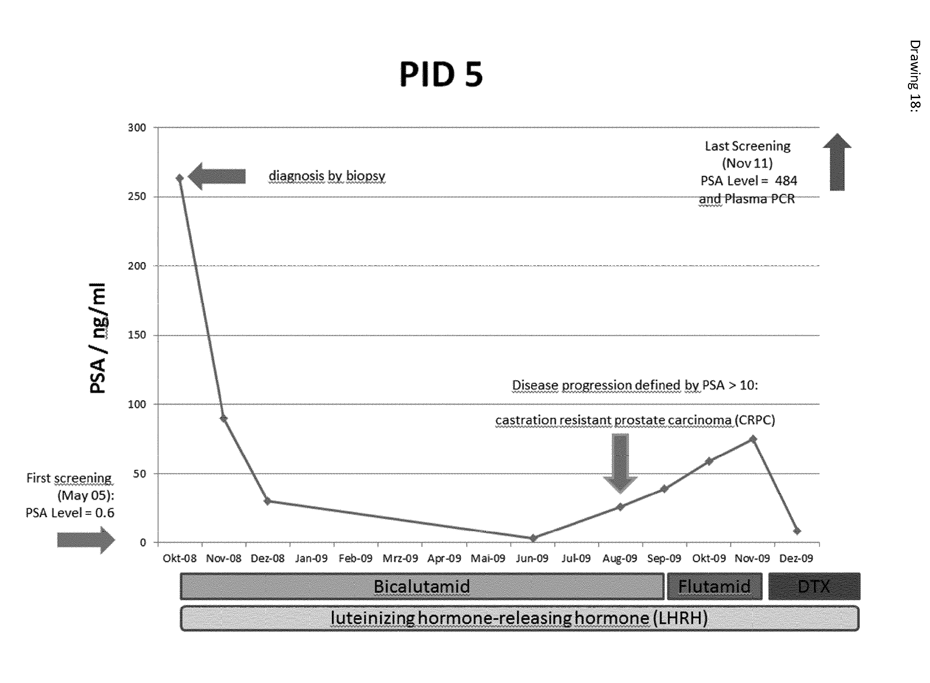

In order to confirm the results of the LNA clamp qRT-PCR analysis of the standard dilutions, the PCR fragments were subsequently sequenced. The sequencing showed that a detection limit of > 1:1,000 and < 1:10,000 was achieved with the aid of the T877A-LNA batch. At a dilution of 1:100, a very weak peak was detectable for the WT in addition to the peak for the mutation (Drawing 14, top diagram). The mutation was still significantly detectable in the 1:1,000 dilution, although the WT signal was half as strong as the signal for the mutation. Almost only WT was detected in the 1:10,000 dilution, although the mutated guanine was still weakly detectable below the WT thymine in the same location as the hotspot peak (Fig. 3.22, top diagram). A detection limit of >= 1:1,000 was achieved with the aid of the W741C-LNA batch (significant peak for adenine, see Drawing 14, bottom diagram). At a dilution of 1:10,000 of the mutation in the WT, it was no longer possible to detect the mutation at all (single peak for WT). The effectiveness of the sensitive detection of the point mutations using LNAs was demonstrated by means of a comparison with samples without the addition of LNAs. Under these conditions, only the WT signal was detected in each dilution (data not shown). In general, it can be said that the W741C LNA blocker displayed lower blocker efficiency than the T877A LNA blocker based on the theoretically determined detection limit. The resolution of the 1:1,000 dilution was also better in the T877A than in the W741C batch in the standard dilutions. The theoretical detection limits were found to correlate with the sequence analyses. The 1:1,000 dilution was resolved in both batches using an electropherogram. In the T877A batch, a weak peak was still detectable for the mutation even at a dilution of 1:10,000. The electropherogram sequence is reversely complementary to the coding sequence of the human AR. In order to illustrate the sequencing results, the base substitutions of the two codons have been put together again below:
W741C hotspot (highlighted in **bold** ):
   3'-**G**GT-5' → 3'-**T**GT-5'
   5'-**C**CA-3' → 5'-**A**CA-3' (reverse compl., electropherogram sequence)
T877A hotspot (highlighted in **bold** ):
   3'-TC**A**-5' → 3'-TC**G**-5'
   5'-AG**T**-3' → 5'-AG**C**-3' (reverse compl., electropherogram sequence)

Drawing 14 shows the sequencing results for W741C/T877A-LNA batches in standard dilutions. Top diagram: Part of the electropherogram showing the sequence covering the T877A blocker (16 nt) from a dilution of 1:10-1:10,000 (mutant DNA in WT DNA) with the addition of T877A-LNA in a final concentration of 0.5 µM. Bottom diagram: Part of the electropherogram showing the sequence covering the W741C blocker (10 nt) from a dilution of 1:10-1:10,000 (mutant DNA in WT DNA) with the addition of W741C-LNA in a final concentration of 3 µM. The base calling sequence has been shown using WT and mutation + LNA for illustration (above the electropherogram). It is reversely complementary to the template strand (3'-5'; see top sequence). Codon 741 and 877 (point mutations) have been placed inside a frame. Codons 740-742 and 876-878 have also been highlighted. The hotspot has been marked with an arrow. The horizontal grey arrow marks the decrease in the concentration of the mutation. Cytosine, adenine,thymine, and guanine.

### 4: Analysis of clinical samples for Bicalutamide resistance (W741C) using LNA blockers

The established LNA assay was analyzed for applicability for the sensitive detection of castration resistance-relevant point mutations in the human AR of prostate cancer patients. Seventy percent of the patients were under or had received Bicalutamide therapy. Only 10% were under or had received hydroxyflutamide therapy. Results are based onto W741C batch. In connection with this, the aim was to identify potential Bicalutamide resistance in clinical samples. Measurements were performed on double samples with a volume of 5 µl from the patient and subject samples used in each, either with or without addition of W741C LNA blocker. Because there was a significant variation between the clinical samples in terms of their initial DNA concentration (0.02-21.64 ng/µl), it was not possible to calculate a blocker efficiency (delta-delta Ct). A reference sample with the same concentration was lacking for this. Six negative controls (subjects) were also run for the batch. From preliminary tests with plasma from subjects, it was possible to identify by means of qRT-PCR analysis that approximately 0.1 ng/µl (0.06 - 0.17 ng/µl) cfDNA was used on average (data not shown). In Drawing 15, the delta Ct values for 18 different samples have been applied and compared with the delta Ct value for the average values of the subject samples. An imaginary threshold was established on the basis of the positive and negative standard deviations of the subject sample (reference). Delta Ct values of the clinical samples falling below the negative threshold were considered as putatively positive for a tryptophane (W) to cysteine (C) base substitution in codon 741. They were therefore classified as Bicalutamide-resistant. This concerned Patient Identity (PID) 4, 5 and 11. These were present at the time of blood sampling during (possibly PID 11) or after Bicalutamide therapy (PID 4 and PID 5) (see Drawing 15). None of the treatment-naive patients (white bars, Drawing 15) displayed a delta CT value below the lower threshold.
Drawing 15 shows the determination of Bicalutamide resistance of clinical samples using W741C-LNA blockers. The delta Ct values of the clinical and subject samples calculated using the difference in the Ct values with and without addition of W741C-LNA were applied. As a reference, the delta Ct value was calculated from the average values for the subject samples (n=6) and applied with the standard deviation. The horizontal lines indicate the upper and lower imaginary threshold as the positive and negative standard deviation. Delta Ct values falling below the negative threshold were classified as putatively positive for the W741 C base substitution. Black bars correspond to patients under Bicalutamide therapy and white bars represent treatment-naive patients. The gray column corresponds to the arithmetic mean for the subject samples. All values were determined in duplicate.

### 5: Sequence analysis for verifying the results (W741C)

The sequencing of the samples for confirming a base substitution and putative Bicalutamide resistance has been summarized in Drawing 16. Sequencing was performed once only because of the small volume of plasma. Without the addition of 3 µM W741C-LNA (final concentration), only a WT signal was detected (data not shown). When W741C-LNA was added, the base substitution was confirmed in the result obtained previously by means of W741C-LNA clamp qRT-PCR in Patient PID 5 (see Drawing 16, PID 5 highlighted in dark grey). The wild-type cytosine base in the same position is more weakly detectable. No clear statement could be made regarding the PID 4 patient sample which also yielded a positive result in the qRT-PCR. PID 11 was classified as positive for the base substitution by means of qRT-PCR. A nucleotide mutation in codon W741C was found in the sequencing. The explanation for this was another single nucleotide polymorphism (SNP) in codon 741 (W741R). If an SNP occurred within the LNA blocker sequence or within the triplet sequence to be analyzed, the LNA was bound to the complementary sequence with a lower affinity than the one calculated theoretically. The consequence was weaker inhibition of the sequence by the LNA (smaller delta Ct value), which resulted in the detection of another mutation in the same codon.

Drawing 16 shows sequence analysis of PID 1 - PID 19 with addition of W741C-LNA. For additional annotation, see Drawing 14. Codon 741 is inside a gray frame. Base substitution occurred in PID 5 (CCA -> ACA, highlighted in red). The position of the possible base substitution (hotspot) was marked with a black arrow. SNPs resulting from automatic base calling have been represented beneath the electropherogram. The theoretical sequence has been noted above the electropherogram; differences from automatic base calling have been represented in dark grey letters.

The above results demonstrate the level of sensitivity with which mutated sequences can be amplified in a high WT DNA background using the LNA clamp PCR method. In summary, it can be said that the results of the *in vitro* tests with the standard dilutions in the presence and absence of the probe correlated with the results of the clinical samples with regard to the sensitive detection of point mutations. It was also possible to detect unknown SNPs sensitively using this method.

Our work focused on the applicability of ctDNA as a blood-based biomarker. Specifically, the LNA clamp qRT PCR method for the sensitive detection of point mutations in the human AR was established and validated. Patients with advanced prostate cancer are treated with the aid of antiandrogens (Bicalutamide, hydroxyflutamide). These inhibit the AR competitively with testosterone [93]. Point mutations in the LBD of the AR (T877A and W741C) lead to the administered antiandrogens having agonist activity and thus to unhindered tumor growth [99, 101, 106]. The difficulty posed by detection lies in the ratio of mutant to WT DNA (between 1:100 and 1:10,000). For the establishment of an assay, primers and LNA blockers were designed as a first step. For optimal blocker efficiency, different annealing and elongation temperatures were tested, and titrations were performed with different gDNA and LNA concentrations. For the T877A assay, the optimal blocker efficiency was achieved with 25 ng gDNA and a final LNA concentration of 0.5 µM (delta-delta Ct of approximately 13; AT: 60 °C; ET: 72 °C). In the W741C assay, the final LNA concentration was increased to 3 µM. The blocker efficiency was also increased by the addition of MgCl2 (in a final concentration of 4 mM) (delta-delta Ct of approximately 11; AT: 55 °C; ET: 74°C). In order to determine the detection limit at which both assays still distinguished between mutant DNA and WT background, standard dilutions (1:10 - 1:10,000) were used. Both assays still identified mutant DNA in a background of >= 1,000 x WT. Sequencing confirmed the results. The applicability of the W741C assay and thus the detection of Bicalutamide resistance was then tested on clinical samples. 3/19 patients were classified as mutation-positive (PID 4, 5, 11). Sequencing showed that PID 5 actually had a W741C mutation. The sequence of PID 4 could not be evaluated. PID 11 had a different SNP in codon 741 which also resulted in a lower delta Ct value. While the clinical significance of this SNP is unclear, this result also demonstrates that our invention can detect deviations from wild type. It was shown that the LNA clamp qRT-PCR was capable of detecting mutated sequences in a background of >= 1,000 x WT DNA not only *in vitro* but also *in vivo.* Because this method has sufficient potential for the sensitive detection of point mutations, it could be used for diagnostic purposes in the future.

**Primers**

| | |
|---|---|
| AR1_FOR_W741C | GACCAGATGGCTGTCATTCA |
| AR5_REV_W741C | AAAGCCGCCTCATACTGGAT |
| AR1_FOR_T877A | CCCTACAGATTGCGAGAGAG |
| AR5_REV_T877A | CAAGGCACTGCAGAGGAGTA |
| LNA-blockers | |
| W741C_LNA | T*-C*-C*-T-G*-G*-A-T*-G*-G*/3InvdT/ |
| T877A_LNA | C*-A*-G*-T-T-C*-A*-C*-T*-T-T-T-G*-A-C*-C*/3InvdT/ |

### 6. Patient PSA level: PID 5 treated with Bicalutamid (Casodex©)

The diagram shows the development of the prostate cancer of the patient over time as indicated by his PSA levels.

PSA values were determined in a clinical routine lab using the Hybritech assay (Beckman-Coulter). The patient presented in Oct. 2008 (approx. 3.5 yrs. after last screening) with a PSA beyond 250 ng/ml.

Biopsy and further differential diagnosis confirmed presence of prostate cancer already metastasized to the bones .Therefore, combined antiandrogen therapy was commenced, LHRH to reduce testosterone production and bicalutamid to block the androgen receptor in the tumor cells. Response to treatment was initally checked monthly. When PSA levels decreased towards normal, Bicalutamid was continued for another six months. Because resistane is known to evolve over time, PSA measurements where then again done more frequently, even though in June 2009 the marker had returned to normal, eight months into the therapy. Indeed, a development to castration resistant prostate cancer was diagnosed in Sept. 2009, because two consecutive PSA measurements had shown an increase beyond normal values. Therefore, androgen receptor blocking treatment was switched from Bicalutamid to Flutamid.

However, further increases in PSA under flutamid indicated no success of this therapy, therefore the patient was switched to general chemotherapy using doxotaxel (DTX) to which the tumor responded. After several cycles, patient was switched to further therapies (not shown).

Blood used to obtain plasma for PCR analysis was drawn in Nov. 2011, 26 months after end of Bicalutamid therapy. Our molecular analysis revealed the mechanism of resistance to Bicalutamid in a low invasive fashion.

Drawing 18: X axis is the Time axis, from Oct 2008 to Dec 2009. Y axis is the PSA level axis.

References
[1] G. J. Kello_ and C. C. Sigman, _Cancer biomarkers: selecting the right drug for the right patient.,_ Nature reviews. Drug discovery, vol. 11, pp. 201_214, Feb. 2012.
[2] L. A. Devriese, E. E. Voest, J. H. Beijnen, and J. H. M. Schellens, _Circulating tumor cells as pharmacodynamic biomarker in early clinical oncological trials,_ Cancer Treatment Reviews, vol. 37, no. 8, pp. 579_589, 2011.
[5] C. L. Sawyers, _The cancer biomarker problem.,_ Nature, vol. 452, pp. 548_52, Apr. 2008.
[7] Medizinreport, _Personalisierte Medizin in der Onkologie: Fortschritt oder falsches Versprechen,_ Deutsches Arzteblatt, vol. 108, no. 37, pp. 1904_1909, 2011.
[8] M. Cristofanilli,_Circulating tumor cells, disease progression, and survival in metastatic breast cancer.,_ The New England Journal of Medicine, vol. 351, pp. 781_791, June 2004.
[9] S. Nagrath, L. Sequist, S. Maheswaran, and D. Bell, _Isolation of rare circulating tumour cells in cancer patients by microchip technology,_ Nature, vol. 450, no. 7173, pp. 1235_1239, 2007.
[10] F. Diehl, M. Li, D. Dressman, Y. He, D. Shen, S. Szabo, L. a. Diaz, S. N. Goodman, K. a. David, H. Juhl, K. W. Kinzler, and B. Vogelstein, _Detection and quanti_cation of mutations in the plasma of patients with colorectal tumors.,_ Proceedings of the National Academy of Sciences of the United States of America, vol. 102, pp. 16368_ 73, Nov. 2005.
[11] X. Wang, J. Yu, A. Sreekumar, S. Varambally, R. Shen, D. Giacherio, R. Mehra, J. E. Montie, K. J. Pienta, M. G. Sanda, P. W. Kanto_, M. a. Rubin, J. T. Wei, D. Ghosh, and A. M. Chinnaiyan, _Autoantibody signatures in prostate cancer.,_ The New England journal of medicine, vol. 353, pp. 1224_35, Sept. 2005.
[61] H. Schwarzenbach, D. S. B. Hoon, and K. Pantel, _Cell-free nucleic acids as biomarkers in cancer patients.,_ Nature reviews. Cancer, vol. 11, pp. 426_37, June 2011.
[62] P. Mandel and P. Métais, _Les acides nucléiques du plasma sanguin chez l'homme.,_ Comptes Rendus de l'Académie des Sciences, vol. 142, pp. 241_-243, 1948.
[63] S. Jahr, H. Hentze, S. Englisch, D. Hardt, F. O. Fackelmayer, and R.-d. Hesch, _DNA Fragments in the Blood Plasma of Cancer Patients : Quantitations and Evidence for Their Origin from Apoptotic and Necrotic Cells DNA Fragments in the Blood Plasma of Cancer Patients : Quantitations and Evidence for Their Origin from Apoptotic and Necr,_ Cancer Research, vol. 61, pp. 1659_1665, 2001.
[65] C. Alix-Panabières, H. Schwarzenbach, and K. Pantel, _Circulating tumor cells and circulating tumor DNA.,_ Annual review of medicine, vol. 63, pp. 199_215, Jan. 2012.
[66] C. Roth, K. Pantel, V. Müller, B. Rack, S. Kasimir-Bauer, W. Janni, and H. Schwarzenbach, _Apoptosis-related deregulation of proteolytic activities and high serum levels of circulating nucleosomes and DNA in blood correlate with breast cancer progression.,_ BMC cancer, vol. 11, p. 4, Jan. 2011.
[67] M. Fleischhacker and B. Schmidt, _Circulating nucleic acids (CNAs) and cancer_a survey.,_ Biochimica et biophysica acta, vol. 1775, pp. 181_232, Jan. 2007.
[68] A. Bendich, T. Wilczok, and E. Borenfreund, _DNA as a Possible Factor in Oncogenesis, _Science, vol. 148, no. 3668, pp. 374_376, 1965.
[69] P. Laktaniov and S. Tamkovich, _Cell Surface Bound Nucleic Acids: Free and Cell Surface Bound Nucleic Acids in Blood of Healthy Donors and Breast Cancer Patients, _ Annals of the New York Academy of Sciences, vol. 9, no. 1022, pp. 221_227, 2004.
[70] H. Schwarzenbach, C. Alix-Panabières, I. Müller, N. Letang, J.-P. Vendrell, X. Rebillard, and K. Pantel, _Cell-free tumor DNA in blood plasma as a marker for circulating tumor cells in prostate cancer.,_ Clinical cancer research : an o_cial journal of the American Association for Cancer Research, vol. 15, pp. 1032_8, Feb. 2009.
[71] E. A. Punnoose, S. Atwal, W. Liu, R. Raja, B. M. Fine, B. G. M. Hughes, R. J. Hicks, G. M. Hampton, L. C. Amler, A. Pirzkall, and M. R. Lackner, _Evaluation of Circulating Tumor Cells and Circulating Tumor DNA in Non-Small Cell Lung Cancer: Association with Clinical Endpoints in a Phase II Clinical Trial of Pertuzumab and Erlotinib.,_ Clinical cancer research : an o_cial journal of the American Association for Cancer Research, vol. 18, Apr. 2012.
[72] B. Schmidt, S. Weickmann, C. Witt, and M. Fleischhacker, _Integrity of cell-free plasma DNA in patients with lung cancer and nonmalignant lung disease.,_ Annals of the New York Academy of Sciences, vol. 1137, pp. 207_13, Aug. 2008.
[73] W. Pao and M. Ladanyi, _Epidermal growth factor receptor mutation testing in lung cancer: searching for the ideal method.,_ Clinical cancer research : an o_cial journal of the American Association for Cancer Research, vol. 13, pp. 4954_5, Sept. 2007.
[80] C. Rhodes, C. Honsinger, D. Porter, and G. Sorenson, _Analysis of the allele-speci_c PCR method for the detection of neoplastic disease,_ Diagnostic Molecular Pathology, vol. 6, no. 1, pp. 49_57, 1997.
[81] H. Orum, P. E. Nielsen, M. Egholm, R. H. Berg, O. Buchardt, and C. Stanley, _Single base pair mutation analysis by PNA directed PCR clamping.,_ Nucleic acids research, vol. 21, pp. 5332_6, Nov. 1993.
[82] M. Behn, C. Thiede, a. Neubauer, W. Pankow, and M. Schuermann, _Facilitated detection of oncogene mutations from exfoliated tissue material by a PNA-mediated 'enriched PCR' protocol.,_The Journal of pathology, vol. 190, pp. 69_75, Jan. 2000.
[83] P. L. Dominguez and M. S. Kolodney, _Wild-type blocking polymerase chain reaction for detection of single nucleotide minority mutations from clinical specimens.,_ Oncogene, vol. 24, pp. 6830_4, Oct. 2005.
[84] A. Senescau, A. Berry, and F. Benoit-Vical, _Use of a locked-nucleic-acid oligomer in the clamped-probe assay for detection of a minority Pfcrt K76T mutant population of Plasmodium falciparum,_ Journal of Clinical Microbiology, vol. 43, no. 7, pp. 3304_ 3308, 2005.
[85] J.-D. Luo, E.-C. Chan, C.-L. Shih, T.-L. Chen, Y. Liang, T.-L. Hwang, and C.-C. Chiou, _Detection of rare mutant K-ras DNA in a single-tube reaction using peptide nucleic acid as both PCR clamp and sensor probe.,_ Nucleic acids research, vol. 34, p. e12, Jan. 2006.
[87] S. Obika, D. Nanbu, Y. Hari, K.-i. Morio, Y. In, T. Ishda, and T. Imanishi, _Synthesis of 2'-O,4'-C-Methyleneuridine and -cytidine. Novel Bicyclic Nucleosides Having a Fixed C3-endo Sugar Puckering,_ Tetrahedron Letters, vol. 38, no. 50, pp. 8735_8738, 1997.
[88] A. A. Koshkin, S. K. Singh, P. Nielsen, V. K. Rajwanshi, R. Kumar, M. Meldgaard, E. Olsen, and J. Wengel, _LNA (Locked Nucleic Acids): Synthesis of the Adenine, Cytosine, Guanine, 5-Methylcytosine, Thymine and Uracil Bicyclonucleoside Monomers, Oligomerisation, and Unprecedented Nucleic Acid Recognition,_Tetrahedron, vol. 54, pp. 3607_3630, 1998.
[89] Y. You, B. G. Moreira, M. a. Behlke, and R. Owczarzy, _Design of LNA probes that improve mismatch discrimination.,_ Nucleic acids research, vol. 34, p. e60, Jan. 2006.
[95] H. Faus and B. Haendler, _Post-translational modi_cations of steroid receptors.,_ Biomedicine & pharmacotherapy = Biomédecine & pharmacothérapie, vol. 60, pp. 520_8, Nov. 2006.
[96] S. Kato, T. Sato, T. Watanabe, S. Takemasa, Y. Masuhiro, F. Ohtake, and T. Matsumoto, _Function of nuclear sex hormone receptors in gene regulation.,_ Cancer chemotherapy and pharmacology, vol. 56, pp. 4_9, Nov. 2005.
[97] I.J. McEwan,_The Nuclear Receptor Superfamily,_ Methods in Molecular Biology, vol. 505, pp. 3_18, 2009.
[98] P. M. Matias, P. Donner, R. Coelho, M. Thomaz, C. Peixoto, S. Macedo, N. Otto, S. Joschko, P. Scholz, a. Wegg, S. Bäsler, M. Schäfer, U. Egner, and M. a. Carrondo, _Structural evidence for ligand speci_city in the binding domain of the human androgen receptor. Implications for pathogenic gene mutations.,_The Journal of biological chemistry, vol. 275, pp. 26164_71, Aug. 2000.
[99] J. Veldscholte, C. Ris-Stalpers, G. Kuiper, and G. Jenser, _A Mutation in the Ligand Binding Domain of the Androgen Receptor of Human LNCaP Cells A_ects Steroid Binding Characteristics and Response to Anti-Androgens,_ Biochemical and Biophysical Research Communications, vol. 173, no. 2, pp. 534_540,1990.
[100] C.-Y. Chang, P. J. Walther, and D. P. Mcdonnell, _Glucocorticoids Manifest Androgenic Activity in a Cell Line Derived from a Metastatic Prostate Cancer Glucocorticoids Manifest Androgenic Activity in a Cell Line Derived from a Metastatic Prostate Cancer 1,_ Cancer Research, vol. 61, pp. 8712_8717, 2001.
[101] J. Veldscholte, C. a. Berrevoets, a. O. Brinkmann, J. a. Grootegoed, and E. Mulder, _Anti-androgens and the mutated androgen receptor of LNCaP cells: di_erential e_ects on binding a_nity, heat-shock protein interaction, and transcription activation., _ Biochemistry, vol. 31, pp. 2393_9, Mar. 1992.
[102] J. Gaddipati, D. McLeod, and H. Heidenberg, _Frequent detection of codon 877 mutation in the androgen receptor gene in advanced prostate cancers,_ Cancer research, vol. 54, no. 11, pp. 2861_2864, 1994.
[103] M. Taplin, G. Bubley, Y. Ko, E. Small, and M. Upton, _Selection for androgen receptor mutations in prostate cancers treated with androgen antagonist,_ Cancer research, vol. 59, no. 11, pp. 2511_2515,1999.

Sequence 'W741C_LNA': Invalid character 'x' at location 1.
Sequence 'W741C_LNA': Invalid character 'x' at location 2.
Sequence 'W741C_LNA': Invalid character 'x' at location 3.
Sequence 'W741C_LNA': Invalid character 'x' at location 5.
Sequence 'W741C_LNA': Invalid character 'x' at location 6.
Sequence 'W741C_LNA': Invalid character 'x' at location 8.
Sequence 'W741C_LNA': Invalid character 'x' at location 9.
Sequence 'W741C_LNA': Invalid character 'x' at location 10.
Sequence 'W741C_LNA': Invalid character 'x' at location 11.
Sequence 'T877A_LNA': Invalid character 'x' at location 1.
Sequence 'T877A_LNA': Invalid character 'x' at location 2.
Sequence 'T877A_LNA': Invalid character 'x' at location 3.
Sequence 'T877A_LNA': Invalid character 'x' at location 6.
Sequence 'T877A_LNA': Invalid character 'x' at location 7.
Sequence 'T877A_LNA': Invalid character 'x' at location 8.
Sequence 'T877A_LNA': Invalid character 'x' at location 9.
Sequence 'T877A_LNA': Invalid character 'x' at location 13.
Sequence 'T877A_LNA': Invalid character 'x' at location 15.
Sequence 'T877A_LNA': Invalid character 'x' at location 16.
Sequence 'T877A_LNA': Invalid character 'x' at location 17.
Sequence 'W741C_LNA_np': Invalid character 'x' at location 1.
Sequence 'W741C_LNA_np': Invalid character 'x' at location 2.
Sequence 'W741C_LNA_np': Invalid character 'x' at location 3.
Sequence 'W741C_LNA_np': Invalid character 'x' at location 5.
Sequence 'W741C_LNA_np': Invalid character 'x' at location 6.
Sequence 'W741C_LNA_np': Invalid character 'x' at location 8.
Sequence 'W741C_LNA_np': Invalid character 'x' at location 9.
Sequence 'W741C_LNA_np': Invalid character 'x' at location 10.
Sequence 'T877A_LNA_np': Invalid character 'x' at location 1.
Sequence 'T877A_LNA_np': Invalid character 'x' at location 2.
Sequence 'T877A_LNA_np': Invalid character 'x' at location 3.
Sequence 'T877A_LNA_np': Invalid character 'x' at location 6.
Sequence 'T877A_LNA_np': Invalid character 'x' at location 7.
Sequence 'T877A_LNA_np': Invalid character 'x' at location 8.
Sequence 'T877A_LNA_np': Invalid character 'x' at location 9.
Sequence 'T877A_LNA_np': Invalid character 'x' at location 13.
Sequence 'T877A_LNA_np': Invalid character 'x' at location 15.
Sequence 'T877A_LNA_np': Invalid character 'x' at location 16.

## Claims

1. A PCR method for detecting the presence of at least one nucleotide Mutation in a DNA polynucleotide sequence encoding for a protein or fragments thereof isolated from patient plasma,
**characterized in that**
o a LNA-blocker is used and
o said DNA polynucleotide sequence or fragments thereof is a natural circulating DNA.

2. The PCR method according to claim 1, wherein the protein is an Androgen Receptor (AR).

3. The PCR method according to claim 2 wherein the LNA-blocker is a LNA-oligonucleotide sequence of 6 to 25 bases comprising a mixture of nucleic acid bases and LNA-monomer bases, wherein said LNA-oligonucleotide sequence is complementing/hybridizing a specific DNA sequence of a DNA polynucleotide sequence or fragments thereof, wherein said specific DNA sequence is a Wild type DNA sequence for which one or more Mutations were identified and which result into patient with CRPC.

4. The PCR method according to claim 2 or 3, wherein the Mutation is located at the amino acid position 741 and/or 877 of the Androgen Receptor (AR) sequence.

5. The PCR method according to claims 1 to 4 wherein the LNA-blocker is selected from
SEQ ID 5: W741C_LNA,
SEQ ID 6: T877A_LNA,
SEQ ID 7: W741C_LNA_np and
SEQ ID 8: T877A_LNA_np.

6. The PCR method according to claims 1 to 5 for which a
upstream primer is selected from
SEQ ID 1 W741C and
SEQ ID 3 T877A.

7. The PCR method according to claims 1 to 6 for which a
downstream primer is selected from
SEQ ID 2: AR5_REV_W741C and
SEQ ID 4: AR5_REV_T877A.

8. The PCR method according to claim 1 to 7 wherein the Mutation is found in patient showing a resistance to therapy drugs such as Bicalutamide and/or Flutamide.

9. A LNA-blocker that is defined as a LNA-oligonucleotide sequence of 6 to 25 bases comprising a mixture of nucleic acid bases and LNA-monomer bases wherein said LNA-oligonucleotide sequence is complementing/hybridizing a specific DNA sequence of a DNA polynucleotide sequence encoding for an Androgen Receptor (AR) or fragments thereof wherein the specific DNA sequence is a Wild type DNA sequence for which one or more Mutations were identified and which result into patient with CRPC.

10. the LNA-blocker according to claim 9 selected from
SEQ ID 5: W741C_LNA,
SEQ ID 6: T877A_LNA,
SEQ ID 7: W741C_LNA_np and
SEQ ID 8: T877A_LNA_np.

11. A upstream primer is selected from
SEQ ID 1 W741C and
SEQ ID 3 T877A.

12. A downstream primer is selected from
SEQ ID 2: AR5_REV_W741C and
SEQ ID 4: AR5_REV_T877A.

13. A kit for detecting the presence of at least one nucleotide Mutation in a DNA polynucleotide sequence encoding for a protein or fragments thereof isolated from patient plasma, wherein said DNA polynucleotide sequence or fragments thereof is a natural circulating DNA fragments isolated from patient plasma and the kit comprises a LNA-blocker according to claim 9 or 10.

14. The kit according to claim 13 comprising additionally an upstream primer and wherein the upstream primer and the LNA-blocker are closely localized onto the DNA polynucleotide sequence wherein closely means that the distance between the last nucleic acid base of the upstream primer and the first nucleic acid base of the LNA-blocker is corresponding to a sequence of 3 to 10 nucleic acid bases of the DNA polynucleotide sequence isolated from patient plasma

15. The kit according to claim 13 or 14 wherein the DNA polynucleotide sequence is encoding for an Androgen Receptor (AR).

16. The kit according to claim 13 to 15 wherein the upstream primer is selected from SEQ ID 1 W741C and SEQ ID 3 T877A.

17. The kit according to claim 13 to 16 comprising additionally a downstream primer selected from SEQ ID 2: AR5_REV_W741C and SEQ ID 4: AR5_REV_T877A.
